# EUROPEAN PATENT SPECIFICATION

(11) **EP 4 125 729 B1**
(45) Date of publication and mention of the grant of the patent: **18.02.2026**
(21) Application number: 21733648.6
(22) Date of filing: 27.05.2021
(51) Int. Cl.: A61F 2/24, A61B 17/122

(54) **DEVICES FOR LEAFLET FOLDING OR CAPTURE**
VORRICHTUNGEN ZUM FALTEN ODER FANGEN VON BROSCHÜREN
DISPOSITIFS POUR LE PLIAGE OU LA CAPTURE DE LAMES VALVULAIRES

(30) Priority: 28.05.2020 US 202063031056 P
(43) Date of publication of application: 08.02.2023
(73) Proprietor: Edwards Lifesciences Corporation, Irvine, CA 92614-5688 (US)
(72) Inventor: WITZMAN, Ofir, 30889 Caesarea (IL); ATIAS, Eitan, 30889 Caesarea (IL); COHEN-TZEMACH, Hanoch, 30889 Caesarea (IL); MAIMON, David, 30889 Caesarea (IL)
(74) Representative: Venner, Julia Ann
(86) International application number: PCT/US2021/034409
(87) International publication number: WO 2021/242952

(56) References cited:
- WO-A1-2015/188066
- WO-A1-2018/099484
- US-A1- 2012 215 303

## Description

### CROSS-REFERENCE TO RELATED APPLICATION

This application claims the benefit of U.S. Provisional Application No. 63/031,056, entitled "Methods and Devices for Leaflet Folding or Capture," filed May 28, 2020.

### FIELD

The present disclosure relates to prosthetic heart valves, and to methods and devices for folding or capturing leaflets of existing valvular structures prior to or during implantation of a prosthetic heart valve.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches, such as transcatheter aortic valve replacement (TAVR), are used in various procedures to deliver prosthetic medical devices to locations inside the body that are not readily accessible by surgery or where access without surgery is desirable.

As surgical approaches for valve replacement become available for younger patients, patient lifetime may exceed the corresponding lifetime of the implanted prosthetic valve. Valve-in-valve (ViV) procedures have been developed to mount a new prosthetic valve within the previously-implanted prosthetic valve. However, such procedures may pose a risk of coronary artery obstruction. In particular, the leaflets of the previously-implanted prosthetic valve may block the coronary artery ostia or otherwise inhibit blood flow through the frame of the new prosthetic valve to the coronary artery ostia. A similar problem may occur when a prosthetic valve is percutaneously expanded within a native heart valve, for example, when the native leaflets are displaced outward toward the coronary ostia. Existing methods, which rely on lacerating existing leaflets, require high spatial precision and surgical skill. Moreover, portions of the lacerated leaflet may still act to partially or completely obstruct the coronary ostia.
WO 2018/099484 describes medical devices for endovascularly or transcatheterly replacing a patient's heart valve, including: a delivery system (9) having an entry profile of 30 Fr or less; a self-expandable annulus ring (10) disposed in the delivery system (9) including a self-expandable anchoring and clipping structure and multiple bridges (4) connected to the anchoring and clipping structure; and a replacement heart valve (11) disposed in the delivery system (9) and connected to the bridges (4).
WO 2015/188066 describes a prosthetic heart valve comprising an annular main body, an atrial cap extending radially outwardly from the atrial end of the main body, and a plurality of ventricular anchors extending outwardly from the ventricular end of the main body. Each ventricular anchor can have a proximal end portion connected to the ventricular end, an intermediate portion extending away from the atrial end and then back toward the atrial so as to define a first bend, and a free distal end portion that extends from the intermediate portion. The distal end portion can comprise a first section, a second section, and a second bend between the first and second sections, the first section extending from the intermediate portion in a direction toward the atrial end and radially away from the main body.
US 2012/0215303 describes a replacement heart valve comprising an expandable frame configured to engage a native valve annulus and a valve body mounted to the expandable frame. The valve body can have a plurality of valve leaflets configured to open to allow flow in a first direction and engage one another so as to close and prevent flow in a second direction, the second direction being opposite the first direction.

### SUMMARY

An aspect of the presently claimed invention is set out in the independent claim. Particular embodiments of this aspect are set out in the dependent claims. Any subject matter contained herein that does not fall within the scope of the appended claims is considered as being useful for understanding the invention.

Described herein are embodiments and examples of methods and tools for folding and/or capturing leaflets of a heart valve to avoid, or at least reduce the risk of, obstruction of the coronary ostia. In some embodiments, a part of a leaflet (or parts of leaflets) is captured and held distal to the coronary ostia by one or more external features of a prosthetic heart valve during installation thereof within an existing valvular structure (e.g., native heart valve or a previously implanted prosthetic heart valve). In some examples, a part of a leaflet (or parts of leaflets) is folded upon itself and/or held distal to the coronary ostia by one or more sutures, coupling members, locking members, clip members, and/or spiked grasping members. A prosthetic heart valve can subsequently be installed within the existing valvular structure. The captured and/or folded leaflet parts allow blood to flow to the coronary artery, the ostia of which might otherwise have been blocked by the unmodified leaflet. In other embodiments, a part of a leaflet (or parts of leaflets) is captured or folded prior to or during installation of a prosthetic heart valve within an existing valvular structure (e.g., native heart valve or a previously implanted prosthetic heart valve) at a valve position other than the aortic position (e.g., pulmonary, tricuspid, or mitral valves).

Any of the various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the disclosed technology will become more apparent from the following detailed description, which proceeds with reference to the accompanying figures.

### BRIEF DESCRIPTION OF THE DRAWINGS

FIG. 1 shows a cross-sectional view of a native aortic valve.
FIG. 2A shows a side view of a prosthetic heart valve implanted in the native aortic valve annulus.
FIG. 2B shows the implanted prosthetic heart valve of FIG. 2A as viewed from the ascending aorta.
FIG. 2C shows an exemplary prosthetic valve delivery apparatus that can be used for implanting a prosthetic heart valve, according to one or more embodiments of the disclosed subject matter.
FIGS. 3A-3C illustrate simplified side views during prosthetic valve positioning, expansion, and mounting stages, respectively, for leaflet capture according to a first example.
FIGS. 4A-4D illustrate simplified side views of catheter positioning, leaflet piercing, suture loop formation, and tightening stages, respectively, for leaflet capture according to a second example.
FIG. 4E illustrates a simplified side view of a tightening stage using a sliding member, according to a variation of the second example.
FIG. 4F illustrates a simplified side view of a suture loop locking stage for leaflet capture according to the second example.
FIG. 4G illustrates a simplified side view of a prosthetic heart valve being implanted between the leaflet of the native aortic valve after one or more of the native leaflets have been modified.
FIG. 5A-5H illustrate simplified side views of first catheter positioning, distal-side first anchor formation, proximal-side first anchor formation, second catheter positioning, distal-side second anchor formation, proximal-side second anchor formation, coupling member sliding, and locking stages, respectively, for leaflet capture according to a third example.
FIGS. 6A-6B illustrate a capture device with actuator in an open configuration and in a closed configuration, respectively, according to a fourth example.
FIGS. 6C-6E illustrate simplified side views of capture device positioning, leaflet gathering, and locking stages, respectively, for leaflet capture according to the fourth example.
FIG. 7A illustrates a capture device according to a fifth example.
FIGS. 7B-7D illustrate simplified side views of catheter positioning, capture device partial deployment, and capture device full deployment stages, respectively, for leaflet capture according to the fifth example.
FIG. 8A illustrates a tool for leaflet folding according to a sixth example.
FIG. 8B is a simplified detailed view of the leaflet folding tool of FIG. 8A employing a tip configuration with a spike.
FIGS. 8C-8D are simplified detailed views (side and bottom, respectively) of the leaflet folding tool of FIG. 8A employing a tip configuration with a barbed spike.
FIG. 9A-9F illustrates simplified side views of leaflet folding tool positioning, leaflet engagement, distal positioning of a single leaflet, distal positioning of multiple leaflets, prosthetic heart valve positioning, and prosthetic heart valve expansion, respectively, for leaflet folding according to the sixth example.
FIG. 10A-10B illustrate a deformable capture device in an initial configuration and in a deformed configuration, respectively, according to a seventh example.
FIG. 10C illustrate the deformable capture device with actuator in a closed configuration according to the seventh example.
FIGS. 11A-11E illustrate simplified side views of capture device positioning, leaflet engagement, leaflet gathering, device deformation, and final locked stages, respectively, for leaflet capture according to the seventh example.

### DETAILED DESCRIPTION

### General Considerations

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present, or problems be solved. The technologies from any example can be combined with the technologies described in any one or more of the other examples.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used herein with reference to the prosthetic heart valve assembly and implantation and structures of the prosthetic heart valve, "proximal" refers to a position, direction, or portion of a component that is closer to the user and a handle of the delivery system or apparatus that is outside the patient, while "distal" refers to a position, direction, or portion of a component that is further away from the user and the handle, and closer to the implantation site. The terms "longitudinal" and "axial" refer to an axis extending in the proximal and distal directions, unless otherwise expressly defined.

The terms "axial direction," "radial direction," and "circumferential direction" have been used herein to describe the arrangement and assembly of components relative to the geometry of the frame of the prosthetic heart valve. Such terms have been used for convenient description, but the disclosed embodiments are not strictly limited to the description. In particular, where a component or action is described relative to a particular direction, directions parallel to the specified direction as well as minor deviations therefrom are included. Thus, a description of a component extending along an axial direction of the frame does not require the component to be aligned with a center of the frame; rather, the component can extend substantially along a direction parallel to a central axis of the frame.

As used herein, the terms "integrally formed" and "unitary construction" refer to a construction that does not include any welds, fasteners, or other means for securing separately formed pieces of material to each other.

As used herein, operations that occur "simultaneously" or "concurrently" occur generally at the same time as one another, although delays in the occurrence of operation relative to the other due to, for example, spacing between components, are expressly within the scope of the above terms, absent specific contrary language.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language. As used herein, "and/or" means "and" or "or," as well as "and" and "or."

Directions and other relative references may be used to facilitate discussion of the drawings and principles herein, but are not intended to be limiting. For example, certain terms may be used such as "inner," "outer," "upper," "lower," "inside," "outside,", "top," "bottom," "interior," "exterior," "left," "right," and the like. Such terms are used, where applicable, to provide some clarity of description when dealing with relative relationships, particularly with respect to the illustrated examples. Such terms are not, however, intended to imply absolute relationships, positions, and/or orientations. For example, with respect to an object, an "upper" part can become a "lower" part simply by turning the object over. Nevertheless, it is still the same part and the object remains the same.

The disclosure of numerical ranges should be understood as referring to each discrete point within the range, inclusive of endpoints, unless otherwise noted. Unless otherwise indicated, all numbers expressing quantities of components, molecular weights, percentages, temperatures, times, and so forth, as used in the specification or claims are to be understood as being modified by the term "about." Accordingly, unless otherwise implicitly or explicitly indicated, or unless the context is properly understood by a person of ordinary skill in the art to have a more definitive construction, the numerical parameters set forth are approximations that may depend on the desired properties sought and/or limits of detection under standard test conditions/methods, as known to those of ordinary skill in the art. When directly and explicitly distinguishing embodiments from discussed prior art, the embodiment numbers are not approximates unless the word "about" is recited. Whenever "substantially," "approximately," "about," or similar language is explicitly used in combination with a specific value, variations up to and including 10% of that value are intended, unless explicitly stated otherwise.

### Overview of the Disclosed Technology

Described herein are methods and tools for folding and/or capturing leaflets of a heart valve (e.g., a native heart valve or a previously implanted prosthetic heart valve). In some embodiments, the heart valve is at the aortic position and the folding/capturing is effective to avoid, or at least reduce the risk of, obstructing blood flow to the coronary arteries. In some embodiments, a part of a leaflet (or parts of leaflets) is captured and held distal to (upstream of) the coronary ostia by one or more external features of a prosthetic heart valve during installation thereof within an existing valvular structure (e.g., native heart valve or a previously implanted prosthetic heart valve). Alternatively or additionally, a part of a leaflet (or parts of leaflets) is folded upon itself and held distal to (upstream of) the coronary ostia by one or more sutures, coupling members, and/or capture devices (e.g., locking device, clip member, lowering bar, grasping member). When a new prosthetic heart valve is subsequently installed within the existing valvular structure, the captured and/or folded leaflets of the existing valvular structure are disposed at locations distal to the coronary artery ostia, thereby allowing blood to flow unobstructed to the coronary arteries. In other embodiments, the heart valve is at a position other than the aortic position, e.g., the pulmonary, tricuspid, or mitral positions.

### Examples of the Disclosed Technology

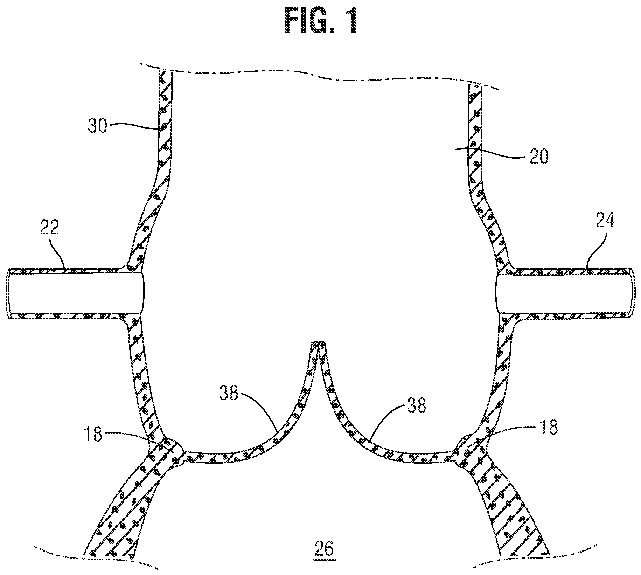

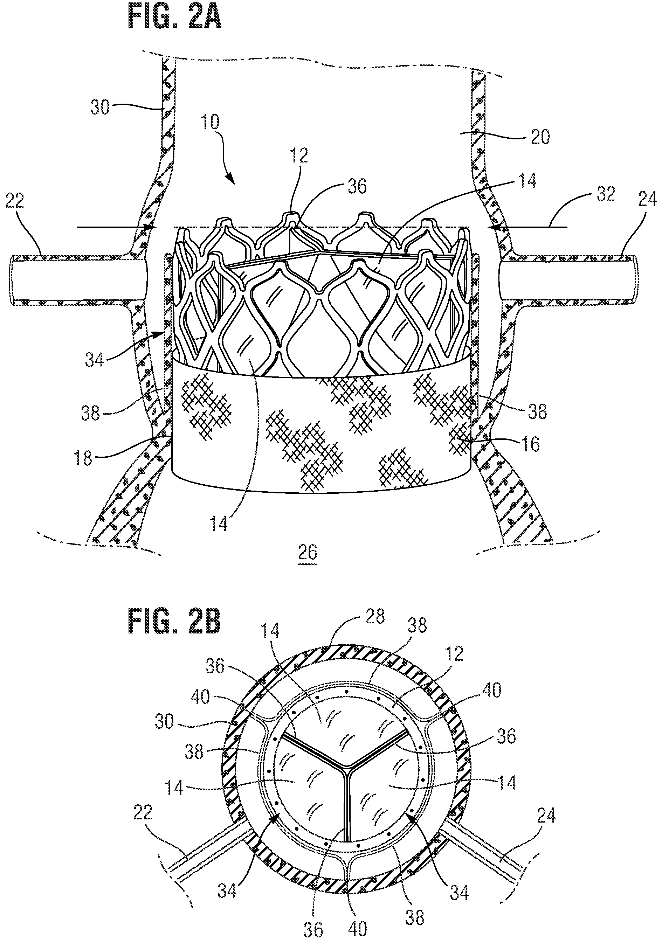

FIG. 1 shows the anatomy of the aortic root of a native valvular structure, which has a plurality of leaflets 38 (e.g., three leaflets, although only two are illustrated in the simplified illustration of FIG. 1) separating the left ventricle 26 from the ascending aorta 20. FIGS. 2A-2B show an exemplary prosthetic heart valve 10 implanted within the aortic annulus 18 of the native valvular structure. The prosthetic heart valve 10 can be radially compressible/expandable between a compressed configuration for delivery into a patient and an expanded configuration for mounting (e.g., as shown in FIG. 2A).

The prosthetic heart valve 10 can include an annular stent or frame 12. The frame 12, or components thereof (e.g., struts and/or fasteners), can be made of any of various suitable plastically-expandable materials (e.g., stainless steel, etc.) or self-expanding materials (e.g., nickel titanium alloy (NiTi), such as nitinol), as known in the art. Suitable plastically-expandable materials that can be used to form the frame 12 include, without limitation, stainless steel, a biocompatible, high-strength alloys (e.g., a cobalt-chromium or a nickel-cobalt-chromium alloys), polymers, or combinations thereof. In particular embodiments, frame 12 is made of a nickel-cobalt-chromium-molybdenum alloy, such as MP35N^{®} alloy (SPS Technologies, Jenkintown, Pennsylvania), which is equivalent to UNS R30035 alloy (covered by ASTM F562-02). MP35N^{®} alloy/UNS R30035 alloy comprises 35% nickel, 35% cobalt, 20% chromium, and 10% molybdenum, by weight.

When constructed of a plastically-expandable material, the frame 12 (and thus the prosthetic valve 10) can be crimped to a radially collapsed configuration on a delivery catheter and then expanded inside a patient by an inflatable balloon or equivalent expansion mechanism. When constructed of a self-expandable material, the frame 12 (and thus the prosthetic valve 10) can be crimped to a radially collapsed configuration and restrained in the collapsed configuration by insertion into a sheath or equivalent mechanism of a delivery catheter. Once inside the body, the prosthetic valve can be advanced from the delivery sheath, which allows the prosthetic valve to expand to its functional size.

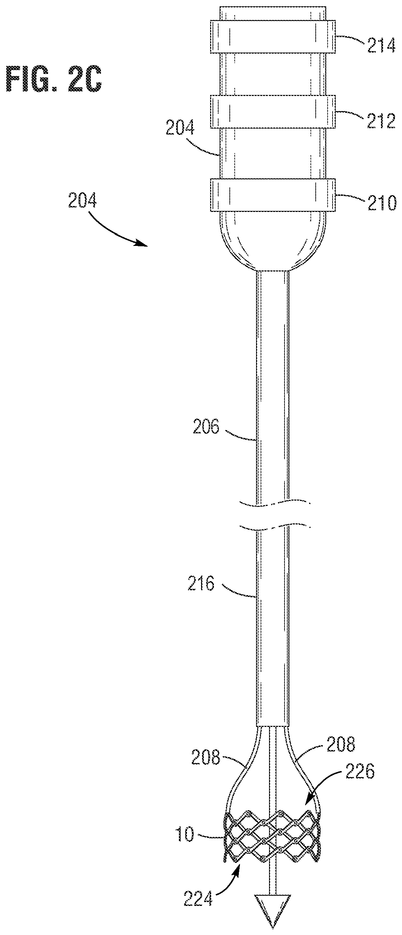

FIG. 2C illustrates an exemplary delivery apparatus 200 adapted to deliver a prosthetic heart valve, such as prosthetic heart valve 10 described herein or any other prosthetic heart valve. The prosthetic valve 10 can be releasably coupled to the delivery apparatus 200, such as via a removable coupling between a distal member of an expansion and locking mechanism of the prosthetic valve 10 and a second actuation member of an actuation assembly of the delivery apparatus 200. The prosthetic valve 10 can include a distal end 224 and a proximal end 226, wherein the proximal end 226 is positioned closer to a handle 204 of the delivery apparatus 200 than the distal end 224, and wherein the distal end 224 is positioned farther from the handle 204 than the proximal end 226. It should be understood that the delivery apparatus 200 can be used to implant prosthetic devices other than prosthetic valves, such as stents or grafts.

The delivery apparatus 200 in the illustrated example generally includes the handle 204, a first elongated shaft 206 (which comprises an outer shaft in the illustrated embodiment) extending distally from the handle 204, at least one actuator assembly 208 extending distally through the outer shaft 206. In some examples, a distal end portion 216 of the shaft 206 can be sized to house the prosthetic valve in its radially compressed, delivery state during delivery of the prosthetic valve through the patient's vasculature. In this manner, the distal end portion 216 functions as a delivery sheath or capsule for the prosthetic valve during delivery.

The at least one actuator assembly 208 can be configured to radially expand and/or radially collapse the prosthetic valve 10 when actuated, and may be removably coupled to the prosthetic heart valve 10. Although the illustrated example shows two actuator assemblies 208 for purposes of illustration, it should be understood that one actuator 208 can be provided for each actuator of the prosthetic valve. For example, three actuator assemblies 208 can be provided for a prosthetic valve having three actuators. In other examples, a greater or fewer number of actuator assemblies can be present. The actuator assemblies 208 can be releasably coupled to the prosthetic valve 10. For example, each actuator assembly 208 can be coupled to a respective actuator of the prosthetic valve 10. Each actuator assembly 208 can comprise a support tube or sleeve and an actuator member. In some examples, the actuator assembly 208 also can include a locking tool. When actuated, the actuator assembly can transmit pushing and/or pulling forces to portions of the prosthetic valve to radially expand and collapse the prosthetic valve. The actuator assemblies 208 can be at least partially disposed radially within, and extend axially through, one or more lumens of the outer shaft 206. For example, the actuator assemblies 208 can extend through a central lumen of the shaft 206 or through separate respective lumens formed in the shaft 206.

The handle 204 of the delivery apparatus 200 can include one or more control mechanisms (e.g., knobs or other actuating mechanisms) for controlling different components of the delivery apparatus 200 in order to expand and/or deploy the prosthetic valve 10. For example, in FIG. 2C, the handle 204 comprises first, second, and third knobs 210, 212, and 214. The first knob 210 can be a rotatable knob configured to produce axial movement of the outer shaft 206 relative to the prosthetic valve 10 in the distal and/or proximal directions in order to deploy the prosthetic valve from the delivery sheath 216 once the prosthetic valve has been advanced to a location at or adjacent the desired implantation location with the patient's body. For example, rotation of the first knob 210 in a first direction (e.g., clockwise) can retract the sheath 216 proximally relative to the prosthetic valve 10 and rotation of the first knob 210 in a second direction (e.g., counter-clockwise) can advance the sheath 216 distally. In other examples, the first knob 210 can be actuated by sliding or moving the knob 210 axially, such as pulling and/or pushing the knob. In other example, actuation of the first knob 210 (rotation or sliding movement of the knob 210) can produce axial movement of the actuator assemblies 208 (and therefore the prosthetic valve 10) relative to the delivery sheath 216 to advance the prosthetic valve distally from the sheath 216.

The second knob 212 can be a rotatable knob configured to produce radial expansion and/or contraction of the prosthetic valve 10. For example, rotation of the second knob 212 can move the actuator member and the support tube axially relative to one another. Rotation of the second knob 212 in a first direction (e.g., clockwise) can radially expand the prosthetic valve 10 and rotation of the second knob 212 in a second direction (e.g., counter-clockwise) can radially collapse the prosthetic valve 10. In other example, the second knob 212 can be actuated by sliding or moving the knob 212 axially, such as pulling and/or pushing the knob.

The third knob 214 can be a rotatable knob configured to retain the prosthetic heart valve 10 in its expanded configuration. For example, the third knob 214 can be operatively connected to a proximal end portion of the locking tool of each actuator assembly 208. Rotation of the third knob in a first direction (e.g., clockwise) can rotate each locking tool to advance the locking nuts to their distal positions to resist radial compression of the frame of the prosthetic valve. Rotation of the knob 214 in the opposite direction (e.g., counterclockwise) can rotate each locking tool in the opposite direction to decouple each locking tool from the prosthetic valve 10. In other embodiments, the third knob 214 can be actuated by sliding or moving the third knob 214 axially, such as pulling and/or pushing the knob.

Although not shown, in some examples, the handle 204 can include a fourth rotatable knob operative connected to a proximal end portion of each actuator member. The fourth knob can be configured to rotate each actuator member, upon rotation of the knob, to unscrew each actuator member from the proximal portion of a respective actuator. Once the locking tools and the actuator members are uncoupled from the prosthetic valve 10, they can be removed from the patient. Further details regarding construction and operation of a delivery apparatus for delivering and implanting a prosthetic heart valve can be found in U.S. Patent Nos. 8,652,202, 9,339,384, 9,827,093, 9,867,700, 10,076,638, and 10,806,573.

In some embodiments, struts of the frame 12 are pivotable or bendable relative to each other to permit radial expansion and contraction of the frame 12. For example, the frame 12 can be formed (e.g., via laser cutting, electroforming or physical vapor deposition) from a single piece of material (e.g., a metal tube). In other embodiments, the frame 12 can be constructed by forming individual components (e.g., the struts and fasteners of the frame) and then mechanically assembling and connecting the individual components together.

Further details regarding the construction of the frame 12 and the prosthetic heart valve 10 are described in U.S. Patent Application Publication Nos. 2012/0123529, 2018/0153689, 2018/0344456, 2019/0060057, 2019/0365530, 2020/0188099, and 2020/0390547, and International Application Publication Nos. WO-2020/081893 and WO-2021/003167.

The frame 12 can have a first axial end and a second axial end. In the depicted embodiment, the first axial end (e.g., facing the ascending aorta 20 near sinotubular junction level 32) can be an outflow end, and the second axial end (e.g., facing the left ventricle 26 near aortic annulus 18) can be an inflow end. In some embodiments, the outflow end can be coupled to a delivery apparatus for delivering the prosthetic valve to the implantation site. Alternatively, the prosthetic valve 10 can be radially crimped on an inflatable balloon of a delivery apparatus for delivery to the implantation site. Implanting the prosthetic heart valve 10 within the native aortic valve can be via a transfemoral, retrograde delivery approach. Thus, in the delivery configuration of the prosthetic heart valve, the outflow end is the proximal-most end of the prosthetic valve. In other embodiments, the inflow end can be the proximal-most end of the prosthetic heart valve in the delivery configuration, depending on the particular native valve being replaced and the delivery technique that is used (e.g., transseptal, transapical, etc.). In some cases, the inflow end can be coupled to the delivery apparatus in the delivery configuration.

The prosthetic valve 10 also includes a valvular structure configured for allowing blood flow through the frame 12 in one direction. The valvular structure can be configured to regulate the flow of blood through the prosthetic heart valve 10 from the inflow end to the outflow end. The valvular structure can include, for example, a leaflet assembly formed by one or more leaflets 14 (three leaflets illustrated in FIGS. 2A-2B) made of a flexible material. Adjacent leaflets 14 can be arranged together to form commissures 36 that are coupled (directly or indirectly) to respective portions of the frame 12, thereby securing at least a portion of the leaflet assembly to the frame 12. The leaflets 14 can be made from, in whole or part, biological material, bio-compatible synthetic materials, or other such materials. Suitable biological material can include, for example, bovine pericardium (or pericardium from other sources). Further details regarding transcatheter prosthetic heart valves, including the manner in which the valvular structure can be coupled to the frame 12 of the prosthetic heart valve 10, can be found, for example, in U.S. Patent Nos. 6,730,118, 7,393,360, 7,510,575, 7,993,394, and 8,652,202, and U.S. Patent Application Publication Nos. 2012/0123529, 2018/0325665, and 2019/0365530.

The prosthetic heart valve 10 can also include one or more skirts or sealing members. For example, the prosthetic heart valve 10 can include an inner skirt mounted on the inner surface (not shown in FIGS. 2A-2B) of the frame 12 and/or an outer skirt 16 mounted on the outer surface of the frame 12. The inner skirt can be a circumferential inner skirt that spans an entire circumference of the inner surface of the frame 12. The inner skirt can function as a sealing member to prevent or decrease perivalvular leakage (e.g., when the valve is placed at the implantation site) and as an attachment surface to anchor a portion of the leaflets 14 to the frame 12. The outer skirt 16 can function as a sealing member by sealing against the tissue of the native valve annulus 18 and helping to reduce paravalvular leakage past the prosthetic valve 10. The inner and outer skirts can be formed from any of various suitable biocompatible materials, including any of various synthetic materials (e.g., polyethylene terephthalate (PET)) or natural tissue (e.g., pericardial tissue). The inner and outer skirts can be mounted to the frame using sutures, an adhesive, welding, and/or other means for attaching the skirts to the frame. Further details regarding the inner and outer skirts and techniques for assembling the leaflets to the inner skirt and assembling the skirts on the frame are disclosed in U.S. Patent Application Publication Nos. 2012/0123529, 2019/0192296, and 2019/0365530, and International Application Publication Nos. WO-2020/159783 and WO-2020/198273.

For an existing implanted prosthetic valve, the valvular structure may naturally degrade over time thereby requiring repair or replacement in order to maintain adequate heart functions. In a Valve-in-Valve (ViV) procedure, a new prosthetic heart valve is mounted within the existing, degrading prosthetic heart valve in order to restore proper function. However, ViV procedures may pose an increased risk of obstruction of the coronary arteries 22, 24. In particular, the mounting of the new prosthetic heart valve within the valvular structure of the existing prosthetic heart valve can displace the leaflets of the existing heart valve outwards, thereby obstructing the ostia of the coronary arteries 22, 24. Moreover, since the leaflets of the existing heart valve are disposed outside the frame of the new prosthetic valve, they may cover external surfaces of the frame, thereby creating a substantially impermeable tubular structure that occludes openings 34 in frame 12. In some patient anatomies (e.g., when the outflow of the valve 10 is at the sinotubular (STJ) level 32 and the diameter of the valve 10 is similar to the STJ diameter such that the frame 12 touches or is very close to the aortic wall 30 at the STJ level 32), the leaflets of the existing valve structure may compromise the ability for future access into the coronary arteries 22, 24 or perfusion through the valve frame 12 to the coronary arteries 22, 24 during the diastole phase of the cardiac cycle. Similar problems may occur in some patient anatomies when a prosthetic heart valve 10 is percutaneously expanded within a native valve, displacing the native leaflets 38 outward toward the coronary ostia.

To avoid obstruction of blood flow to the coronary arteries 22, 24, the leaflets of the existing heart valve (whether a native aortic valve or a previously implanted prosthetic valve) can be captured and/or folded prior to or during implantation of a new prosthetic heart valve within the existing valvular structure. In some embodiments, a new prosthetic heart valve can include one or more components on an exterior of the valve frame. The components can be designed to capture and position one, some, or all of the leaflets of the existing valvular structure away from a level of the coronary arteries as the prosthetic heart valve is installed, thereby maintaining vascular access to the arteries.

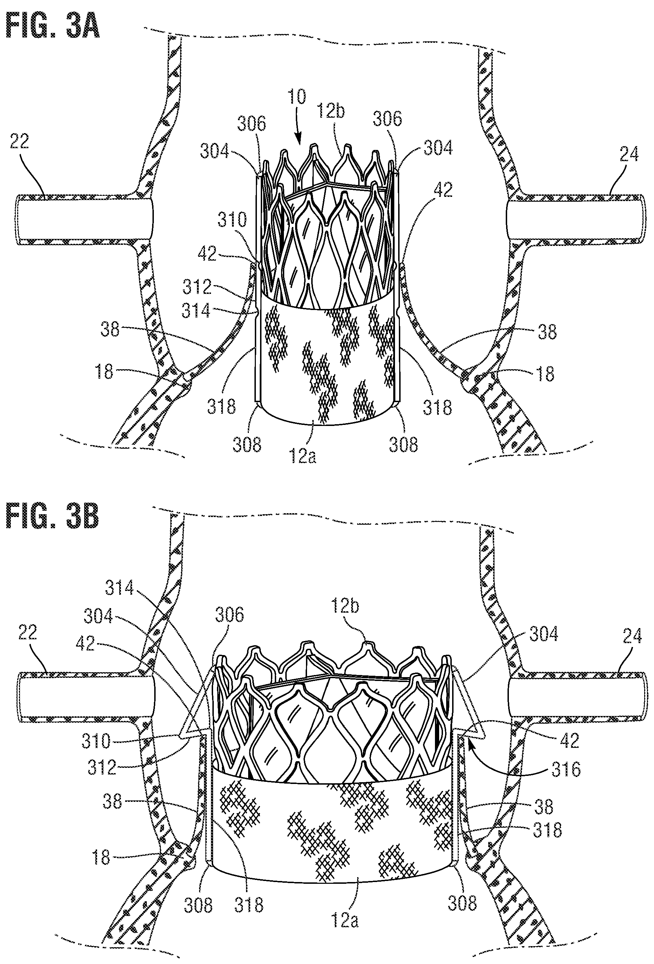

For example, FIGS. 3A-3C illustrate a prosthetic heart valve 10 that includes a leaflet capture member 304 for repositioning one or more leaflets 38 of an existing valvular structure (e.g., the native heart valve in the illustrated example, or a previously implanted prosthetic heart valve in an unillustrated example). The leaflet capture member 304 can be attached to the frame 12 of the prosthetic heart valve 10, for example, via attachment 306 at the proximal end and via attachment 308 at the distal end. For example, attachments 306, 308 can include a suture extending between the respective capture member 304 and the corresponding portion of the frame 12, a rigid attachment member (e.g., a bracket or fastener), and/or a coupling material (e.g., weld, glue, or epoxy). The leaflet capture member 304 may be otherwise free to move independent of the valve frame 12 between the attachments 306, 308.

In the description of the disclosed embodiments, the methods and devices are described in the context of using a retrograde delivery approach to the native aortic valve. As such, the term "proximal end" of the prosthetic valve (or other device) or a component thereof is used to refer to its outflow end and the term "distal end" of the prosthetic valve (or other device) or a component thereof is used to refer to its inflow end. However, it should be noted if delivered in the opposite direction to the aortic valve (e.g., transapically) the outflow end of the prosthetic valve would be the distal end and the inflow end of the prosthetic valve would be the proximal end during delivery. Thus, in the present application, once a prosthetic valve is implanted at the aortic position, the term "proximal end" is intended to mean "outflow end" and the term "distal end" is intended to mean "inflow end". Similarly, the terms "distal side" and "distal" as used herein to describe components or parts of the anatomy are intended to mean "upstream side" and "upstream" while the terms "proximal side" and "proximal" as used herein to describe components or parts of the anatomy are intended to mean "downstream side" and "downstream." Further, any of the methods and devices described herein can be applied to any of the native valves of the heart (the aortic, mitral, tricuspid, and pulmonary valves) or a prosthetic valve previously implanted within any of the native valves of the heart using any known techniques, which can involve approaching a native valve in a retrograde or antegrade direction.

Each leaflet capture member 304 can have a middle portion 312 connected to an upper portion by first inflection portion 310 at its proximal end and connected to a lower portion 318 by second inflection portion 314 at its distal end. For example, each inflection portion 310, 314 can comprise a weakened, notched, or grooved portion or a region with a narrowed cross-section. In some embodiments, the leaflet capture member 304 can be formed as a substantial straight bar that extends along an axial direction of the valve 10. Alternatively, the leaflet capture member 304 can have a cell-like open configuration over at least a portion thereof, for example, the portion between attachment 306 and the first inflection portion 310 so as to allow blood flow therethrough or other access to the coronary arteries 22, 24. The cell-like open configuration of the leaflet capture member 304 may have a similar configuration to and be aligned with the cell-like open configuration of the frame 12 of the prosthetic heart valve 10 (e.g., similar in size/shape to and/or aligned with opening 34 in frame 12).

In some embodiments, each leaflet capture member 304 can be disposed at a location along a circumferential direction of the valve 10 corresponding to the commissures of the valvular structure of the prosthetic valve, for example, on a side of the frame 12 directly opposite where a commissure attaches to the frame 12. In other embodiments, the leaflet capture members 304 can be disposed at other locations, for example, at locations along a circumferential direction of the valve 10 that correspond with locations of the ostia of the coronary arteries 22, 24 once the valve 10 is implanted. Although two leaflet capture members 304 are illustrated, fewer or additional members 304 are also possible, for example, a leaflet capture member 304 corresponding to each leaflet of the existing valvular structure. In addition, although illustrated as an axially-extending bar in the figures, other shapes for the leaflet capture member 304 are also possible according to one or more contemplated embodiments.

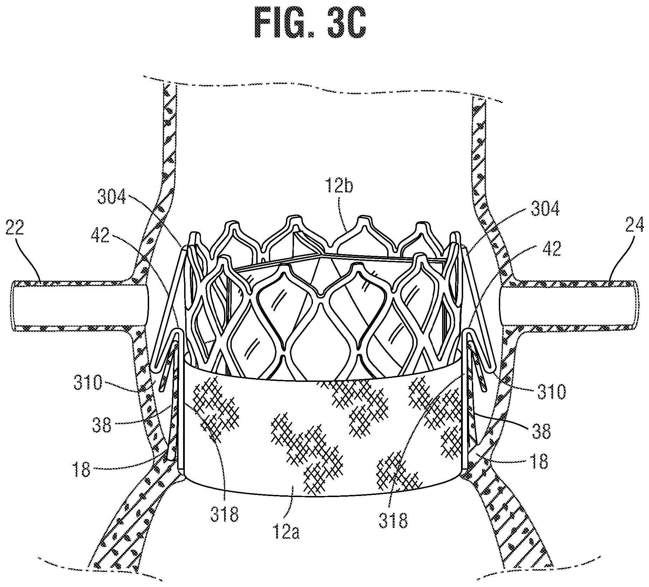

FIG. 3A shows the heart valve 10 in the initial crimped state within the existing valvular structure (e.g., the native aortic valve). The leaflet capture member 304 can have a substantially straight longitudinal configuration and can be positioned to contact a portion of the leaflets (e.g., a free end 42 of leaflet 38). As the heart valve 10 is expanded radially, the heart valve 10 contracts along its axis, thereby compressing the leaflet capture members 304 via attachments 306, 308. As attachments 306. 308 approach each other during expansion of the valve 10, axial pressure can be exerted on the leaflet capture member 304. Under application of the axial pressure, inflection portion 310 may be constructed to deflect radially outward and inflection portion 314 may be constructed to deflect radially inward, as illustrated in FIG. 3B. The folding of the leaflet capture member 304 about inflection points 310, 314 can form a pocket 316 between the middle portion 312 and the lower portion 318 of the leaflet capture member 304. In particular, the middle portion 312 can rotate about the second inflection portion 314 such that first inflection portion 310 is disposed closer to the distal end 12a of the prosthetic heart valve 10 than the second inflection portion 312. As the heart valve 10 is further expanded into its final configuration with the external wall of the valve frame 12 contacting the surrounding structure (e.g., the native annulus or the frame of the previously installed prosthetic valve), the middle portion 312 approaches the lower portion 318 such that the free end 42 of the leaflet 38 (or other portion of each leaflet) folds upon itself and is captured within pocket 316. The leaflet 38 is thus spaced from the coronary arteries 22, 24 once the heart valve 10 is fully installed, as shown in FIG. 3C.

In some embodiments, a flexible fiber (e.g., suture, wire, or thread) can be used to fold a leaflet upon itself and capture the folded leaflet in a position that avoids obstructing the corresponding coronary ostium. The flexible fiber can pierce the leaflet near a distal end thereof and can pass around the free end of the leaflet. The end of the fiber can be tied to itself with a sliding knot, thereby forming a loop containing therein part of the leaflet that is between the pierced portion and the free end. Sliding the knot in a distal direction (e.g., toward the existing heart valve) can reduce a size of the loop, which causes the leaflet part therein to curl or fold upon itself. Once sufficient compaction of the leaflet is achieved (e.g., reduction in size and/or location that would not obstruct the ostia of the coronary arteries once a prosthetic heart valve is installed within the existing valvular structure), the knot can be locked in place to retain the leaflet therein, and the new prosthetic heart valve can be subsequently installed.

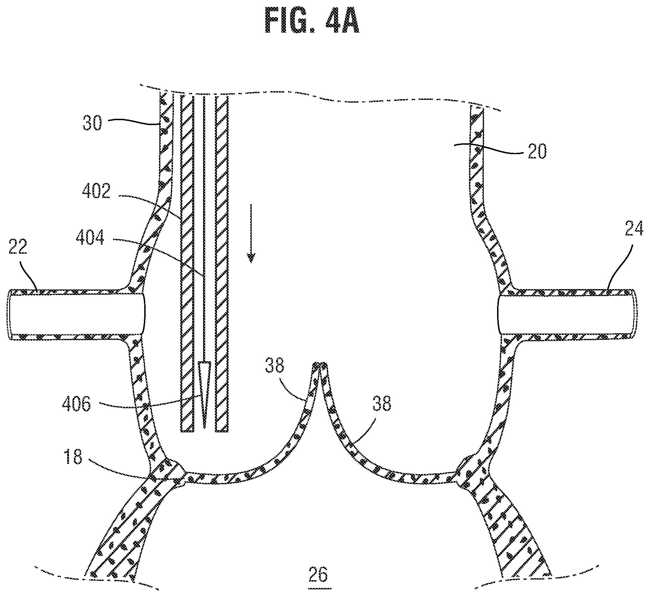

FIGS. 4A-4G illustrate an exemplary method for folding and/or capturing a leaflet using a suture. As shown in FIG. 4A, a delivery catheter 402 can be advanced from the ascending aorta 20 toward one of the leaflets 38 of an existing valvular structure (e.g., a native aortic valve in the illustrated example, or a previously installed prosthetic heart valve in an unillustrated example). For example, the delivery catheter 402 can be positioned with its end facing a first portion of one of the leaflets 38. The first portion can be closer to the valve annulus 18 than the free end 42 of the leaflet 38 and is preferably distal to the ostium of coronary artery 22. In some embodiments, the first portion of leaflet 38 may be circumferentially aligned with the ostium of coronary artery 22 (e.g., aligned or substantially aligned along a same radial vector extending from a center of the existing valvular structure, as viewed from the ascending aorta 20).

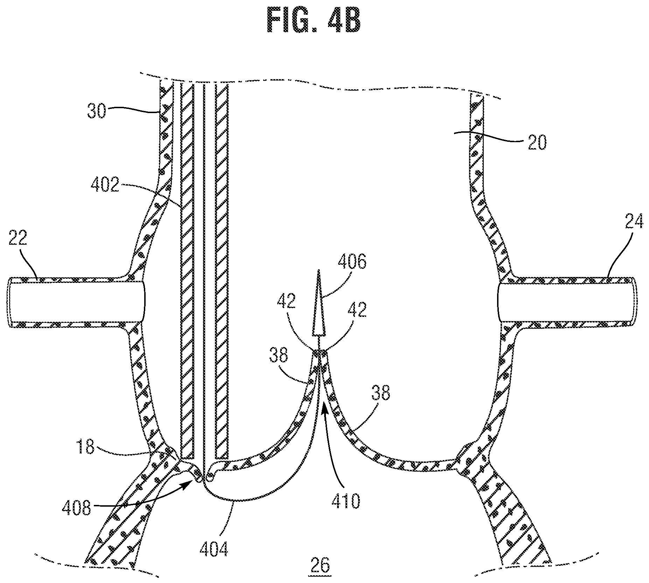

A needle 406 with a suture 404 attached thereto can be disposed within the delivery catheter 402. Once the delivery catheter 402 is disposed adjacent to the first portion of the leaflet 38, the needle 406 can be advanced out of the distal end of the delivery catheter 402 to pierce the first portion 408 of the leaflet 38. The needle 406 with suture 404 thus moves through the first portion 408 to a distal side of the leaflet 38, as shown in FIG. 4B. The suture 404 can then be pulled back to the proximal side of the leaflet 38 by moving the needle 406 through central gap 410 between free ends 42 of the leaflets 38 of the existing valvular structure. A portion 404b of suture 404 that has passed through the central gap 410 can then be tied over a portion 404a of suture 404 that has not passed through the first portion 408 of the leaflet 38.

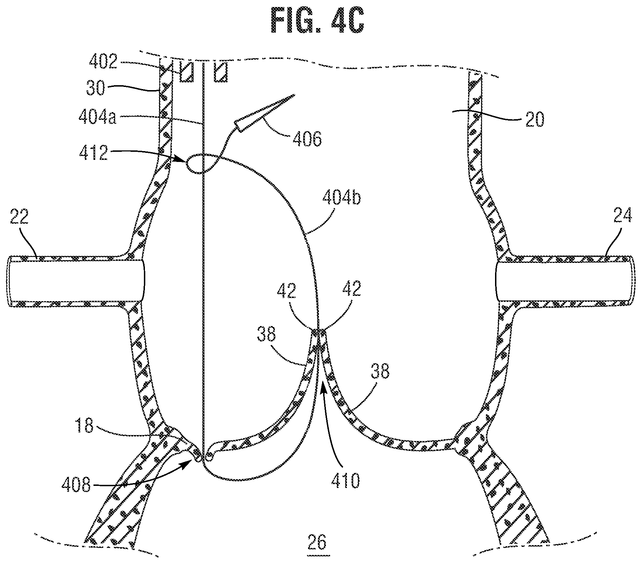

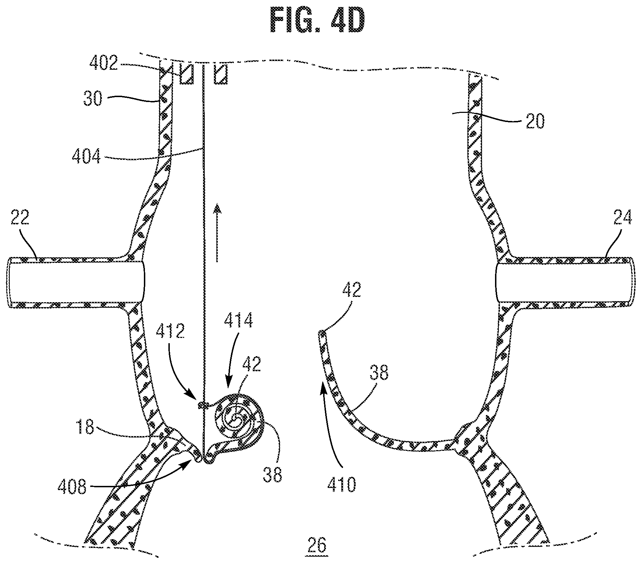

A knot 412 is formed by the tying of portion 404b to portion 404a, as shown in FIG. 4C. Knot 412 and suture portions 404a, 404b define a loop 414 that surrounds part of the leaflet 38 between the first portion 408 and leaflet free end 42. The knot 412 can be formed so as to be slidable along the suture 404. In some embodiments, the configuration of the knot 412 itself allows it to be slidable along the suture 404, for example, by forming a slip knot or other sliding knot. Alternatively or additionally, the knot 412 may be initially formed in a loose state to allow the knot 412 to slide along the suture 404. The knot 412 can thus be slid distally along the suture 404 toward the valvular structure so as to reduce a size of the loop 414. The reduction in the size of the loop 414 causes the part of the leaflet contained therein (i.e., the part of the leaflet 38 between its free end 42 and the pierced first portion 408) to curl or fold upon itself, as shown in FIG. 4D.

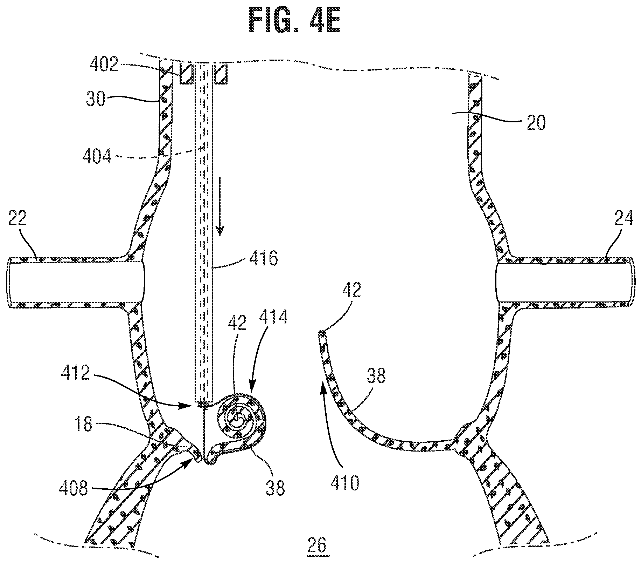

In some embodiments, the sliding of the knot 412 distally toward the valvular structure can be effected by moving at least one of the knot 412 and the suture 404 with respect to the other. For example, the suture 404 can be pulled in the proximal direction, which can tighten the loop 414 and cause the knot 412 to move distally. Alternatively or additionally, the knot 412 can be pushed in the distal direction toward the pierced first portion 408 of the leaflet 38 by a push/assist member, for example, by sliding member 416 as shown in FIG. 4E. Alternatively or additionally, the knot 412 can be held in place by the push/assist member (e.g., a positioning member) as the suture 404 is retracted in the proximal direction. The sliding member 416 can have a through-hole, conduit, or recess through which suture 404 extends and can have a portion (e.g., distal end) constructed to abut the knot 412 when the sliding member 416 is brought in contact therewith. For example, the sliding member 416 can be initially disposed on the suture 404 within the delivery catheter 402, and the sliding member 416 can be sized and shaped to be delivered into the ascending aorta 20 from a distal end of the delivery catheter 402. Alternatively, the push/assist member can be a distal end portion of the delivery catheter 402 rather than a separate member 416.

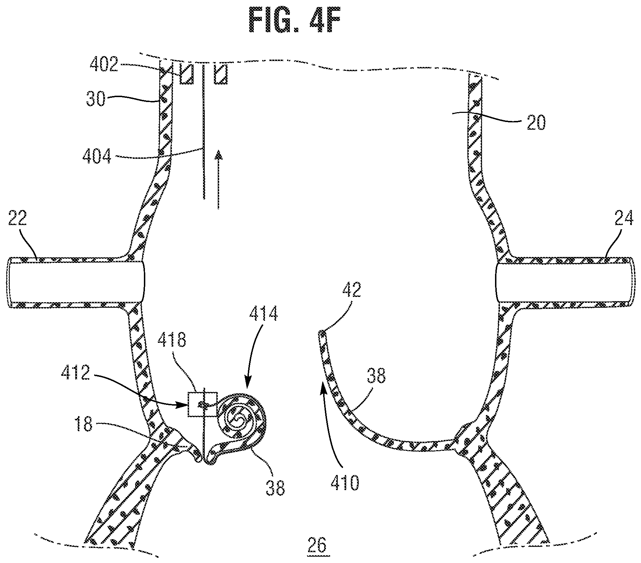

The amount of reduction in the size of the loop 414 can be chosen such that the folded part of the leaflet 38 contained therein is positioned distal to the ostium of coronary artery 22, even when pressed radially outward by subsequent mounting of a prosthetic heart valve within the existing valvular structure. Once sufficient reduction in the size of the suture loop 414 is achieved, the knot 412 can be locked in place, as shown in FIG. 4F. The locking of the knot 412 can ensure that the captured part of the leaflet 38 does not unfold during or after installation of the prosthetic heart valve. For example, the knot 412 can be locked in place by manipulation of the knot itself, such as by tightening the knot 412 and/or bonding, fusing, or encasing the suture portions forming the knot 412. Alternatively or additionally, the knot 412 can be locked in place by a separate physical device 418 that is brought into contact with the knot 412. In some embodiments the sliding member 416 can be used to position the locking device 418 in place with respect to knot 412. The locking device 418 may be constructed to reconfigure between a sliding configuration (e.g., where a through-hole allows relative motion between suture 404 and locking device 418) and a non-sliding configuration (e.g., where the through-hole restricts relative motion between suture 404 and locking device 418, for example, by a member that presses suture 404 into contact with a sidewall of the through-hole). Alternatively or additionally, locking device 418 comprises a suture clip or fastener, for example, as described in U.S. Patent Application Publication Nos. 2018/0177503 and 2020/0000458. With knot 412 locked in place, suture 404 can be cut (e.g., at a location immediately adjacent to the locking device 418 or at another location proximal to a location of the knot 412) and retracted into delivery catheter 402, as shown in FIG. 4F. The cutting of the suture 404 can be made by a cutting tool provided from a distal end of delivery catheter 402 or provided via a separate catheter in the ascending aorta 20. Alternatively, part of delivery catheter 402 (e.g., a distal end of the catheter 402) can include a cutting element for cutting the suture 404.

The suture loop 414 thus folds part of leaflet 38 and captures it distal to coronary artery 22, thereby preventing, or at least reducing the risk of obstruction of the ostium of coronary artery 22. If additional capture/folding of one or more leaflets 38 of the existing valvular structure is desired, for example, to prevent, or at least reduce the risk of, obstruction of the ostium of coronary artery 24, then the techniques of FIGS. 4A-4F can be repeated in a similar manner with respect to the next leaflet 38 using the same delivery catheter 402 or a different delivery catheter. In such repetition, the portion of the next leaflet 38, which is pierced by the needle 406 and suture 404, can be circumferentially aligned with the ostium of coronary artery 24 (e.g., aligned or substantially aligned along a same radial vector extending from a center of the existing valvular structure, as viewed from the ascending aorta 20). Although not discussed in detail above, manipulation of the needle 406 and/or suture 404 within the patient's anatomy, movement of the push/assist member (e.g., sliding member 416) or locking device 418, and/or manipulation of the knot 412 (e.g., to move or lock of the knot) may be performed using any tools employed in laparoscopic and/or transcatheter heart surgeries, such as, but not limited to, knot pushers, suture cutters, manipulators, and the like.

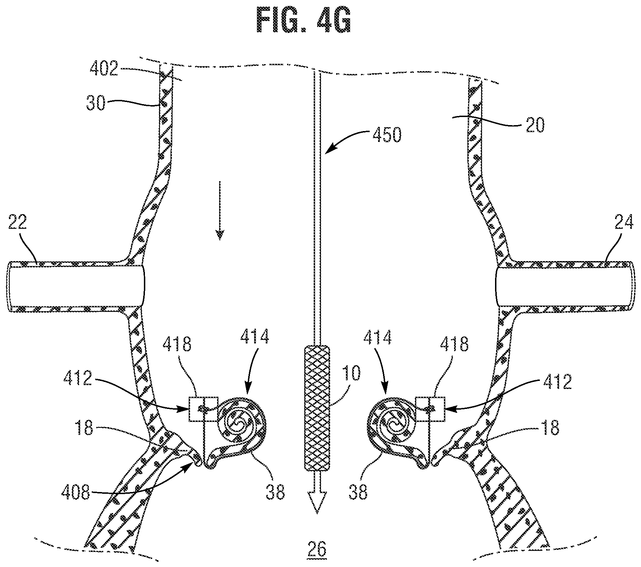

When no further leaflet capture is desired, a new prosthetic heart valve in a crimped state can subsequently be advanced to the existing valvular structure with captured leaflets 38. For example, as depicted in FIG. 4G, a new prosthetic valve 10 (which can be the valve 10 of FIG. 2A or a different prosthetic valve) can be radially crimped on a balloon of a delivery apparatus 450. The delivery catheter 450 can be advanced through the aorta toward the native aortic valve to position the prosthetic valve 10 between the leaflets 38. The new prosthetic valve 10 is then expanded by inflating the balloon of the delivery catheter, such that the captured leaflets 38 are disposed on an external surface of the new valve frame. However, the locked suture loops 414 retain the capture leaflets 38 in a location distal to the coronary arteries 22, 24, thereby allowing blood to flow from the outflow end of the new prosthetic valve to the coronary arteries 22, 24 once the heart valve implantation is completed. Further details of the delivery apparatus and methods for implanting a prosthetic valve using the delivery apparatus are disclosed in U.S. Patent Nos. 7,780,723, 9,061,119, and 9,339,384, and U.S. Patent Application Publication No. 2017/0065415.

In other embodiments, the prosthetic valve 10 can be a self-expandable prosthetic valve that is retained in a radially compressed state within a capsule or delivery sheath of a delivery apparatus, such as disclosed in U.S. Patent Application Publication No. 2014/0343670 and U.S. Patent No. 8,652,202. Once the prosthetic valve is positioned between the leaflets 38, the prosthetic valve can be deployed from the delivery sheath, which allows the prosthetic valve to self-expand to a radially expanded state against the leaflets.

In other embodiments, the prosthetic valve 10 can be a mechanically expandable prosthetic valve that is releasably connected to one or more mechanical actuators of a delivery apparatus, such as disclosed in U.S. Patent Application Publication No. 2018/0153689, U.S. Application No. 62/990,299, and International Application No. PCT/US2020/063104. The prosthetic valve is retained in a radially compressed state by the delivery apparatus (optionally in a delivery sheath) and positioned between the leaflets 38. Once positioned, the prosthetic valve can be deployed from the delivery sheath and radially expanded to a radially expanded state against the leaflets by actuating the one or more mechanical actuators of the delivery apparatus.

In some embodiments, a first member (e.g., suture, wire, thread, or other structure) is tethered to a first portion of the leaflet, and a second member (e.g., suture, wire, thread, or other structure) is tethered to a second portion of the leaflet. The second portion of the leaflet can be near a free end of the leaflet, while the first portion of the leaflet can be near a base or anchor portion of the leaflet (e.g., a distal-most part of the leaflet). The first and second members can be tethered to the respective first and second portions by one or more anchors or plications. By pulling the first and second members toward each other, the second portion of the leaflet is pulled toward the first portion of the leaflet, thereby folding part of the leaflet between the first and second portions. Once sufficient folding of the leaflet is achieved (e.g., reduction in size and/or location that would not obstruct the ostia of the coronary arteries once a prosthetic heart valve is installed within the existing valve structure), the locations of the first and second members with respect to each other can be locked to retain the folded configuration of the leaflet, and the new prosthetic heart valve can be subsequently installed.

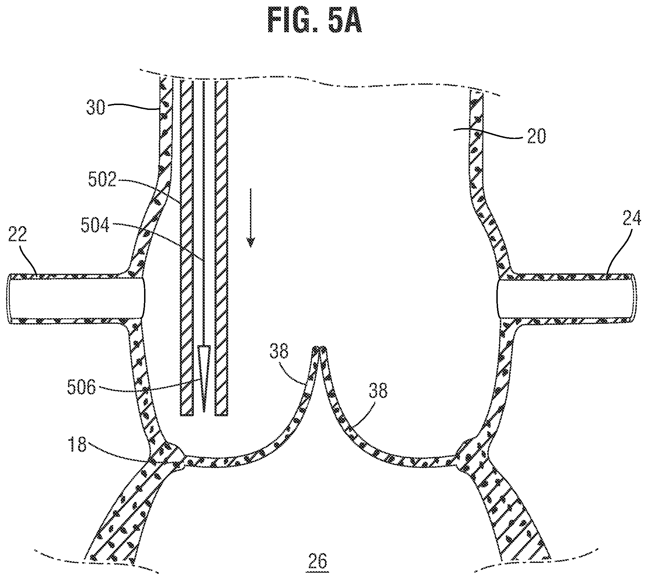

FIGS. 5A-5H illustrate an exemplary method for folding and/or capturing a leaflet using tethered sutures. As shown in FIG. 5A, a delivery catheter 502 can be advanced from the ascending aorta 20 toward one of the leaflets 38 of an existing valvular structure (e.g., a native aortic valve in the illustrated example, or a previously installed prosthetic heart valve in an unillustrated example). For example, the delivery catheter 502 can be positioned with its end facing a first portion of one of the leaflets 38. The first portion can be closer to the valve annulus 18 than the free end 42 of the leaflet 38 and is preferably distal to the ostium of coronary artery 22. In some embodiments, the first portion of leaflet 38 may be circumferentially aligned with the ostium of coronary artery 22 (e.g., aligned or substantially aligned along a same radial vector extending from a center of the existing valvular structure, as viewed from the ascending aorta 20).

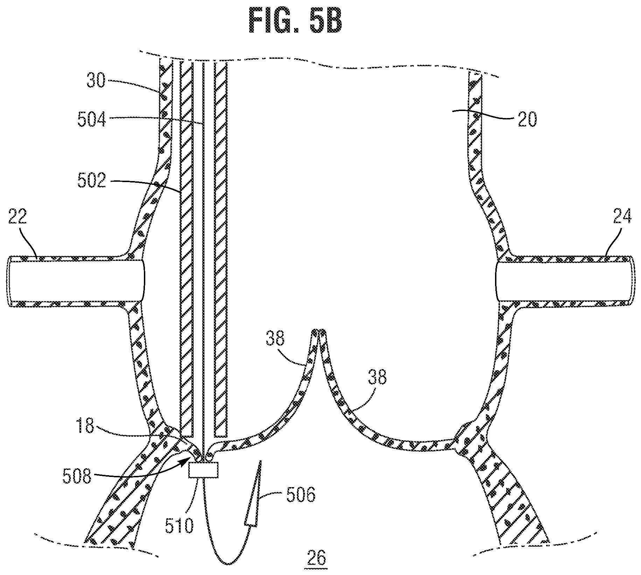

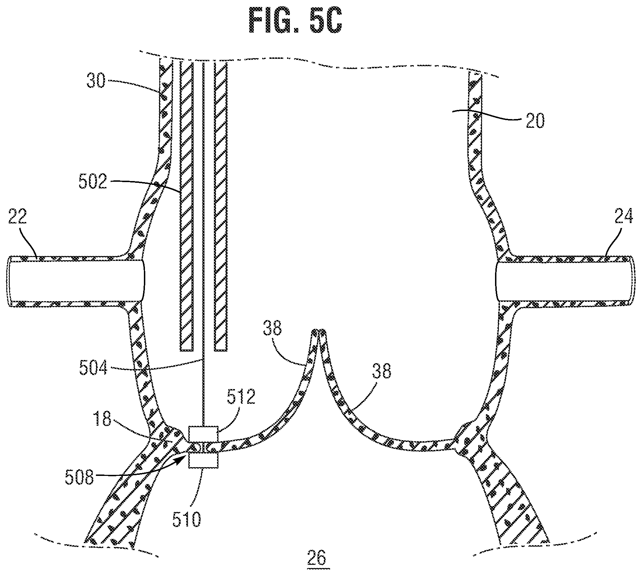

A needle 506 with a first suture 504 attached thereto can be disposed within the delivery catheter 502. Once the delivery catheter 502 is disposed adjacent to the first portion of the leaflet 38, the needle 506 can be advanced out of the distal end of the delivery catheter 502 to pierce the first portion 508 of the leaflet 38. The needle 506 with first suture 504 thus moves through the first portion 508 to a distal side of the leaflet 38, as shown in FIG. 5B. At the first portion 508, at least one anchor can be formed to tether the first suture 504 to the leaflet 38. For example, a distal-side anchor 510 can be formed at first portion 508. Alternatively or additionally, a proximal-side anchor 512 can be formed at first portion 508. As shown in FIG. 5C, the first suture 504 can extend between the distal-side anchor 510 and the proximal-side anchor 512.

For example, each anchor 510, 512 can include a knotted portion of the first suture 504, a piece of cloth or fabric, a plication formed from the leaflet, and/or a locking member. To form the distal-side anchor, the anchoring components (e.g., fabric, clip, etc.) can be passed through the pierced portion of the leaflet with the suture and can be expanded on the distal-side. To form the proximal-side anchor, the anchoring components can be conveyed to the proximal-side surface of the leaflet, for example, by sliding down the suture from the delivery catheter. For example, each anchor 510, 512 can comprise one or more of the suture fasteners or suture clips disclosed in U.S. Patent Application Publication Nos. 2018/0177503 and 2020/0000458. Further details of suture attachment to leaflets can be found in U.S. Patent Application Publication No. 2015/0230919.

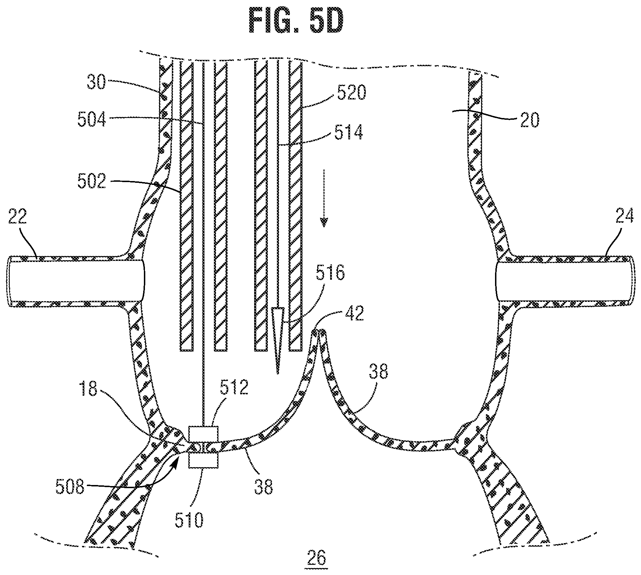

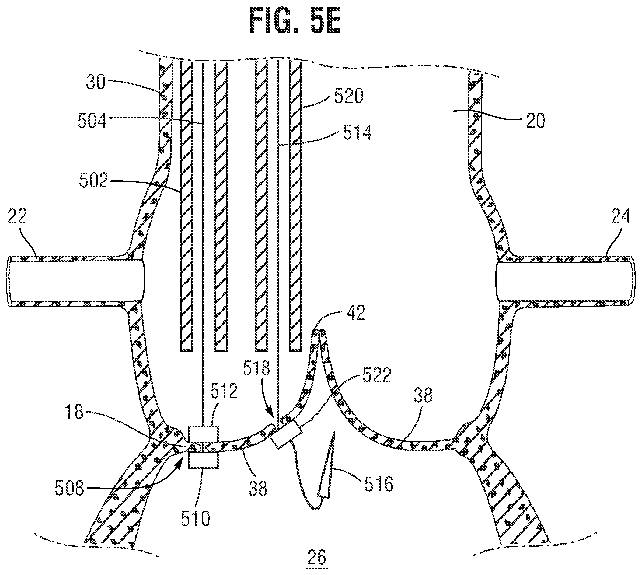

As shown in FIG. 5D, another delivery catheter 520 can be advanced from the ascending aorta 20 and positioned with its distal end facing a second portion of the same leaflet 38. The second portion can be closer to the free end 42 of the leaflet 38 than the first portion 508. In some embodiments, the second portion of leaflet 38 may also be circumferentially aligned with the ostium of coronary artery 22 (e.g., aligned or substantially aligned along a same radial vector extending from a center of the existing valvular structure, as viewed from the ascending aorta 20). Another needle 516 with a second suture 514 attached thereto can be disposed within the delivery catheter 520. Once the delivery catheter 520 is disposed adjacent to the second portion of the leaflet 38, the needle 516 can be advanced out of the distal end of the delivery catheter 502 to pierce the second portion 518 of the leaflet 38. The needle 516 with second suture 514 thus moves through the second portion 518 to the distal side of the leaflet 38, as shown in FIG. 5E.

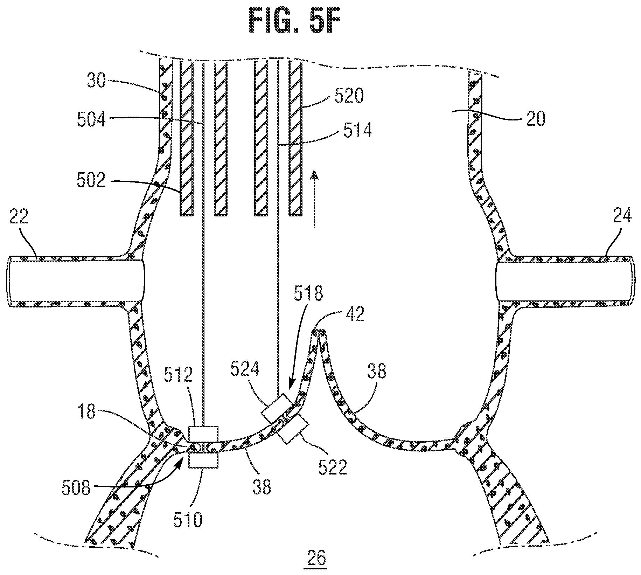

At the second portion 518, at least one anchor can be formed to tether the second suture 514 to the leaflet 38. For example, a distal-side anchor 522 can be formed at second portion 518. Alternatively or additionally, a proximal-side anchor 524 can be formed at second portion 518. As shown in FIG. 5F, the second suture 514 can extend between the distal-side anchor 522 and the proximal-side anchor 524. Similar to anchors 510, 512, each anchor 522, 524 can be a knotted portion of the first suture 504, a plication formed from the leaflet, and/or a locking member and/or can employ one or more of the suture fasteners or suture clips. Although the positioning of delivery catheter 520 and formation of anchors 522, 524 at the second portion 518 has been described after the positioning of delivery catheter 502 and formation of anchors 510, 512 at the first portion 508, such description has been for convenience only. Indeed, the steps described above with respect to FIGS. 5A-5F may occur at the same time or in a different order from that described.

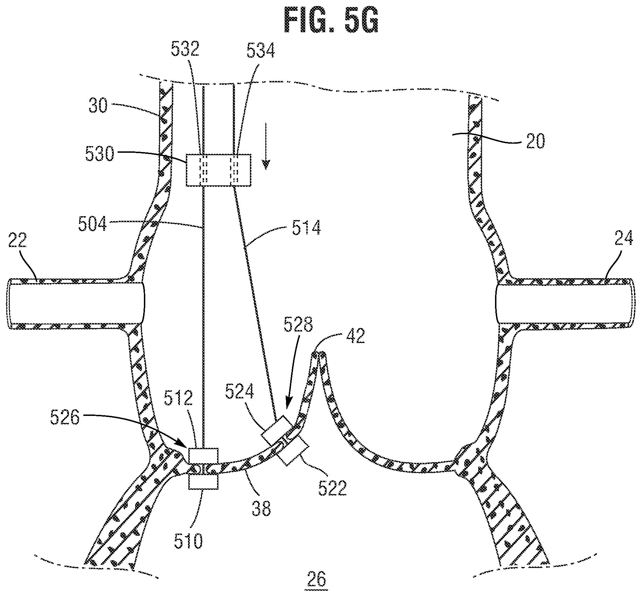

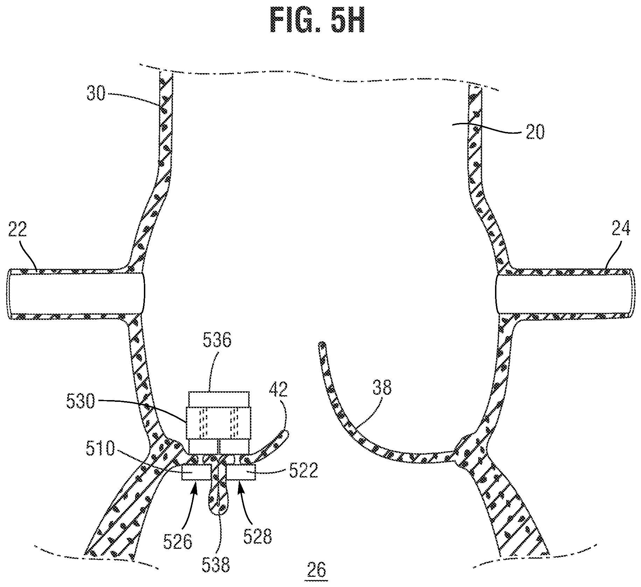

Once sutures 504, 514 are tethered to the first portion 508 and second portion 518 of the leaflet 38 by their respective anchors, the delivery catheters 502, 520 can be retracted and a coupling member 530 can be provided, as shown in FIG. 5G, which can, for example, be delivered via another catheter inserted over the sutures 504, 514 through the ascending aorta. The coupling member 530 can have one or more through-holes, conduits, or recesses through which sutures 504, 514 extend. For example, coupling member 530 can be a sliding member with a pair of through-holes 532, 534. The first suture 504 can extend through the first through-hole 532 while the second suture 514 can extend through the second through-hole 534. A lateral distance between the through-holes 532, 534 can be less than a distance between the first portion 508 and the second portion 518 along a contour of the leaflet 38. Thus, as the coupling member 530 is advanced distally from the ascending aorta 20 toward the leaflet 38, the spacing between the through-holes 532, 534 pulls the first and second portions 508, 518 of the leaflet 38 toward each other, thereby causing the leaflet 38 to fold. In particular, the part 538 of the leaflet between anchored portion 526 (e.g., closest to valve annulus 18) and anchored portion 528 (e.g., closest to free end 42) can fold upon itself to have a corrugated configuration between anchors 510/512 and anchors 522/524, or at least to extend distally away from coronary artery 22, as illustrated in FIG. 5H. Alternatively, the coupling member can be a sliding member with a single through-hole. Both the first and second sutures 504, 514 can extend through the same through-hole. A lateral dimension (e.g., diameter) of a distal end of the through-hole can be less than a distance between the first portion 508 and the second portion 518 along the contour of the leaflet 38. Thus, as the coupling member is advanced toward the leaflet 38, the sidewalls of the through-hole pull the first and second portions 508, 518, of the leaflet 38 toward each other, thereby causing the leaflet 38 to fold.

In some embodiments, the delivery catheter 502 used to tether the first suture 504 to the first portion 508 of the leaflet 38 can be the same as that used to tether the second suture 514 to the second portion 518. For example, after forming the anchors 510, 512 at the first portion 508, the suture 504 can be cut at a location between the distal end of the delivery catheter 502 and the anchor 512, and the severed part of suture 504 retained for subsequent use with a coupling member. The delivery catheter 502 is thus free to reposition with respect to the second portion 518, e.g., as delivery catheter 520 in FIG. 5F. Alternatively, after forming the anchors 510, 512 at the first portion 508, the first suture 504 is maintained extending from the end of the delivery catheter 502 during the repositioning with respect to the second portion 518. The second needle 516 and the second suture 514 can be extended from the delivery catheter 502 while the first suture 504 continues to extend between the first portion 508 and the catheter 502. In such configurations, the delivery catheter 502 may be used as a "single through-hole" coupling member to pull the first portion 508 and the second portion 518 together after forming anchors 522, 524.

Once the coupling member 530 is in contact with the proximal-side anchors 512, 524 and/or when the leaflet has otherwise achieved sufficient folding (the folded leaflet 38 is positioned distal to the ostium of coronary artery 22, even when pressed radially outward by subsequent mounting of a prosthetic heart valve within the existing valvular structure), the coupling member 530 can be locked in place with respect to anchored portions 526, 528, as shown in FIG. 5H. The locking of the coupling member 530 can ensure that the folded part of the leaflet 38 does not unfold during or after installation of the prosthetic heart valve. For example, the coupling member 530 can be locked in place by a separate physical device 536 that is brought into contact with the proximal side of the coupling member 530. Alternatively or additionally, the coupling member 530 may be constructed to be reconfigured between a sliding configuration (e.g., where through-holes 532, 534 allow relative motion between sutures 504, 514 and the coupling member 530) and a non-sliding configuration (e.g., where each through-hole 532, 534 restricts relative motion between sutures 504, 514 and coupling member 530, for example, by a member that presses sutures 504, 514 into contact with a sidewall of the respective through-hole). Alternatively or additionally, the coupling member 530 can be locked in place by manipulation of the sutures themselves. For example, free ends of sutures 504, 514 extending out of the proximal-side of the coupling member 530 can be joined together (e.g., by tying, bonding, fusing, or encasing) in order to lock the coupling member in place.

With coupling member 530 locked in place, each suture 504, 514 can be cut (e.g., at a location immediately adjacent to the locking device 536 or at another location proximal to a location of the coupling member) and retracted proximally, as shown in FIG. 5H. The cutting of each suture 504, 514 can be made by a cutting tool provided from a distal end of either delivery catheter 502, 520 or provided via a separate catheter in the ascending aorta 20. Alternatively, part of one or both of the delivery catheters 502, 520 (e.g., distal ends of catheters 502, 520) can include a cutting element for cutting the respective suture 504, 514.

The anchored portions 526, 528 pulled toward each other thus capture and fold leaflet part 538 and position the leaflet free end 42 distal to coronary artery 22, thereby preventing, or at least reducing the risk of obstruction of the ostium of coronary artery 22. If additional capture/folding of one or more leaflets 38 of the existing valvular structure is desired, for example, to prevent, or at least reduce the risk of, obstruction of the ostium of coronary artery 24, then the techniques of FIGS. 5A-5H can be repeated in a similar manner with respect to the next leaflet 38 using the same delivery catheters 502, 520 or different delivery catheters. In such repetition, the portions of the next leaflet 38, which are pierced by needles 506 and 516 respectively, can be circumferentially aligned with the ostium of coronary artery 24 (e.g., aligned or substantially aligned along a same radial vector extending from a center of the existing valvular structure, as viewed from the ascending aorta 20). Although not discussed in detail above, manipulation of needles 506, 516 and/or sutures 504, 514 within the patient's anatomy, forming or installing anchors 510, 512, 522, 524, and/or movement of the coupling member 530 or locking device 536 may be performed using any tools employed in laparoscopic and/or transcatheter heart surgeries, such as, but not limited to, suture cutters, manipulators, and the like.

When no further leaflet capture/folding is desired, a new prosthetic heart valve in a crimped state can subsequently be advanced to the existing valvular structure with captured leaflets 38 (such as shown in FIG. 4G). The new valve is disposed within the valvular structure and expanded, such that the captured leaflets 38 are disposed on an external surface of the new valve frame. However, the proximated anchored portions 526, 528 retain the leaflet 38 in a position distal to the coronary arteries 22, 24, thereby allowing blood to flow from the outflow end of the new prosthetic valve to the coronary arteries 22, 24 once the heart valve implantation is completed.

In some embodiments, a locking device can be used to capture a leaflet in a folded configuration and at a position that avoids obstructing the corresponding coronary ostium. The locking device can be advanced in an open configuration over a free end of the leaflet. A portion of the locking device or of an actuator thereof can gather part of the leaflet between opposing members of the locking device as the locking device is further advanced over the leaflet. Once a sufficient part of the leaflet has been gathered (e.g., reduction in size and/or location that would not otherwise obstruct the ostia of the coronary arteries once a prosthetic heart valve is installed within the existing valvular structure), the locking device can transition to the closed configuration, where the locking device members approach each other to contact and retain the gathered part of the leaflet therebetween, and the new prosthetic heart valve can be subsequently installed.

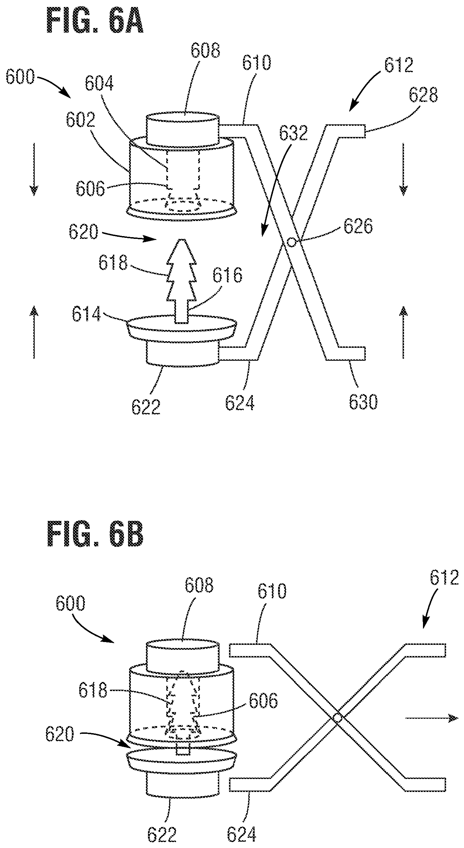

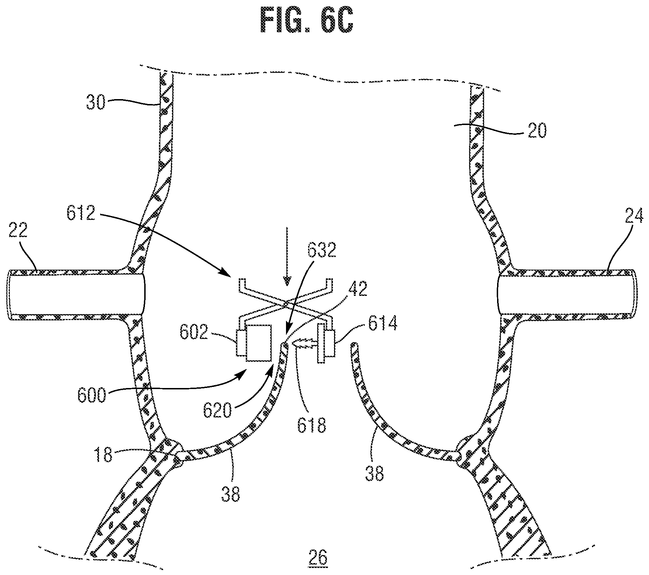

FIGS. 6A-6B illustrate an exemplary locking device, and FIGS. 6C-6E illustrate an exemplary method for folding and/or capturing a leaflet using the exemplary locking device. In particular, FIG. 6A illustrates the locking device 600 in an open configuration, and FIG. 6B illustrates the locking device 600 in the closed configuration. The locking device 600 can have a first member 602 and a second member 614. In the open configuration, the first member 602 is spaced from the second member 614 to define a gap 620 therebetween. In the closed configuration, the first member 602 and the second member 614 approach each other to eliminate, or at least reduce a size of, the gap 620. The first member 602 can be constructed as a female member, for example, having one or more recesses 604. The second member 614 can be constructed as a male member, for example, one or more projections or tines 616. Each projection 616 corresponds to one of the recesses 604, with the projection 616 being spaced from and aligned with the corresponding recess 604 in the open configuration, and the projection 616 being inserted into and in contact with a portion of the corresponding recess 604 in the closed configuration. Each projection 616 can have a sharp tip constructed to pierce a leaflet within gap 620 as the locking device 600 transitions from the open configuration to the closed configuration.

The projection 616 and the recess 604 can be constructed with one or more snap-fit features, for example, to prevent removal of projection 616 from recess 604 once the projection 616 has been inserted into the recess 604 in the closed configuration. For example, projection 616 may include a barbed portion 618, and recess 604 may include a ridge 606 therein. As the locking device 600 transitions to the closed configuration, the projection 616 is inserted into the recess 604 such that an edge of barbed portion 618 abuts ridge 606 to resist withdrawal of the projection 616 from the recess 604. In some embodiments, the projection 616 includes multiple barbed portions, for example, to accommodate a variation in thicknesses for the gathered portion of the leaflets between the first and second members 602, 614. In addition to snap-fit locking features between the recess 604 and projection 616, or in place thereof, the locking device 600 can include one or more external locking features, such as, but not limited, a linear ratchet disposed on external portions of members 602, 614, for example, adjacent to gap 620. In such an alternative configuration, the linear ratchet can be used as a gathering member, as described further below.

Although only one projection 616 and one recess 604 is shown in FIGS. 6A-6B, embodiments of the disclosed subject matter are not limited thereto. Rather, the second member 614 can have multiple separate projections 616, and the first member 602 can have multiple corresponding recesses 604. Moreover, although each member 602, 614 is illustrated with only one of the projection 616 or recess 604, members 602, 614 may include both in some embodiments. For example, the second member 614 can have one or more recesses in addition to projection 616, and the first member 602 can have one or more projections in addition to recess 604. In embodiments with multiple projections and rejections, one, some, or all of the projection-recess pairs can include snap-fit features.

Transition of the locking device 600 from the open configuration to the closed configuration can be effected by an actuator. For example, the actuator can be a scissor-style actuator 612, such as that shown in FIG. 6A. The actuator 612 can have a first arm 610 attached to a coupling portion 608 of the first member 602 and a second arm 624 attached to a coupling portion 622 of the second member 614. The first arm 610 and the second arm 624 can be coupled together at hinge 626, which allows arms 610, 624 to rotate with respect to each other. Application of a compressive force at ends 628, 630, which force pushes the ends together, causes the arms 610, 624 to pivot about hinge 626 and thereby urge first member 602 and second member 614 toward each other. The actuator arms 610, 624 can be releasably attached to respective coupling portions 608, 622, such that the actuator 612 can be removed from the locking device 600 after it is placed in the closed configuration, for example, as shown in FIG. 6B. Other styles of actuators are also possible according to one or more contemplated embodiments. Indeed, any actuator capable of operating within the ascending aorta of a patient and of controllably moving the first and second members 602, 614 toward each other while maintaining alignment between recess 604 and projection 616 can be used.

In some embodiments, the actuator 612 defines a region 632 adjacent to gap 620 that is designed to gather part of the leaflet into gap 620 as the locking device 600 is moved from the free end of the leaflet toward the valve annulus. For example, region 632 can be formed by the portion of arms 610, 624 at the hinge 626 that face gap 620. Alternatively or additionally, a separate structure (e.g., a gathering member) can be disposed between the actuator 612 and gap 620 and can be used to gather the leaflet. For example, the gathering member may include a suture, wire, or thread extending between coupling portions 608, 622 in region 632. In another example, gather member may be a bar extending between actuator arms 610, 624 in region 632, with slots that allow the actuator arms 610, 624 to move unrestricted with respect to each other.

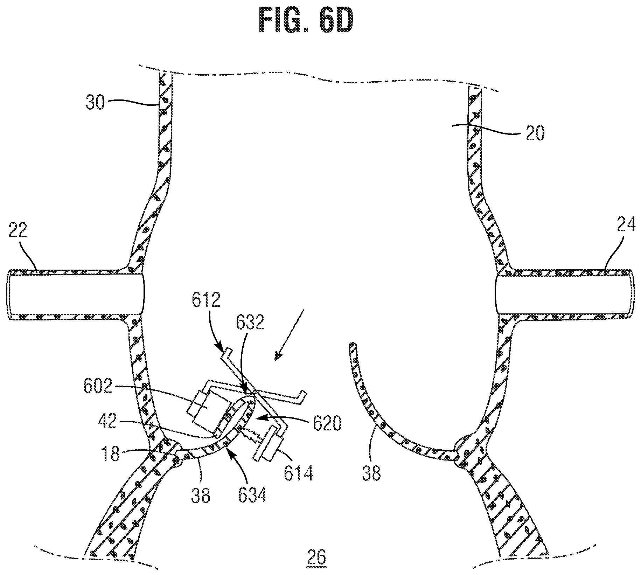

As shown in FIG. 6C, the locking device 600, with actuator 612, can be advanced in the open configuration from the ascending aorta 20 to be positioned with respect to one of the leaflet 38 of an existing valvular structure (e.g., a native aortic valve in the illustrated example, or a previously installed prosthetic heart valve in an unillustrated example). For example, the free end 42 of leaflet 38 can be arranged within gap 620 between first member 602 and second member 614. The locking device 600, with actuator 612, can then be further advanced along the leaflet 38, as shown in FIG. 6D. For example, as the locking device 600 is moved toward the valve annulus 18, the region 632 adjacent the hinge 626 of actuator 612 abuts the free end 42 of the leaflet 38 and causes it to fold upon itself, such that folded portion 634 of leaflet 38 is gathered within gap 620. Alternatively or additionally, a gathering member between the gap 620 and region 632 can contact the free end 42 of the leaflet 38 in order to cause the leaflet 38 to fold upon itself as the locking device 600 is advanced.

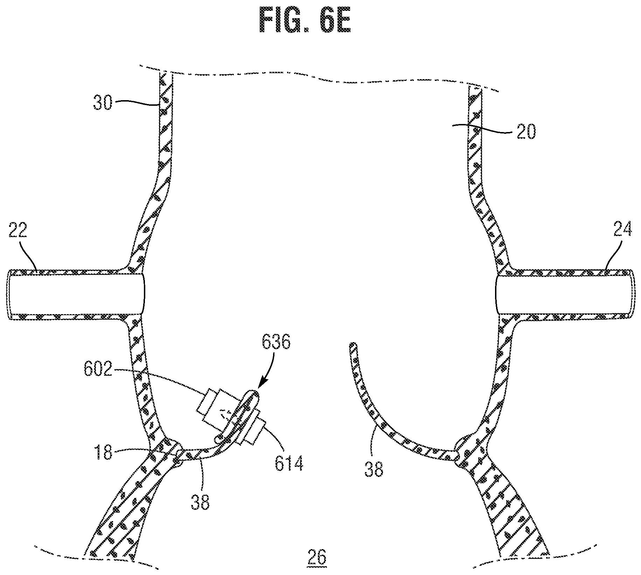

The amount of locking device advancement can be chosen such that the proximal folded edge 636 of the leaflet 38 is positioned distal to the ostium of coronary artery 22, even when pressed radially outward by subsequent mounting of a prosthetic heart valve within the existing valvular structure. Once the locking device 600 has been sufficiently advanced over the leaflet, the actuator 612 can be actuated to transition the locking device 600 to the closed configuration, as shown in FIG. 6E. The projection of the second member 614 thus moves toward the first member 602, thereby piercing the folded portions of leaflet in gap 620, before being inserted into corresponding recess of the first member 602. Transition to the closed configuration can further lock the first and second members together, for example, via one or more of the snap-fit features described above. The locking of the locking device can ensure that the captured part 634 of the leaflet 38 does not unfold during or after installation of the prosthetic heart valve. The actuator 612 can thus be released from the locking device 600 and can be used to subsequently install a locking device on another leaflet or otherwise retrieved from the patient.

The installation of the locking device 600 thus folds part of leaflet 38 and captures it distal to coronary artery 22, thereby preventing, or at least reducing the risk of obstruction of the ostium of coronary artery 22. If additional capture/folding of one or more leaflets 38 of the existing valvular structure is desired, for example, to prevent, or at least reduce the risk of, obstruction of the ostium of coronary artery 24, then the techniques of FIGS. 6C-6F can be repeated in a similar manner with respect to the next leaflet 38 using another locking device and the same actuator 612 or a different actuator. Although not discussed in detail above, manipulation of the locking device 600 and/or actuator 612 within the patient's anatomy, actuation of actuator 612, and/or decoupling of actuator 612 may be performed using any tools employed in laparoscopic and/or transcatheter heart surgeries.

When no further leaflet capture is desired, a new prosthetic heart valve in a crimped state can subsequently be advanced to the existing valvular structure with leaflets 38 captured by locking devices 600 (such as shown in FIG. 4G). The new valve is disposed within the valvular structure and expanded, such that the captured leaflets 38 are disposed on an external surface of the new valve frame. However, the locking devices 600 retain the captured leaflets 38 in a location distal to the coronary arteries 22, 24, thereby allowing blood to flow from the outflow end of the new prosthetic valve to the coronary arteries 22, 24 once the heart valve implantation is completed.

In some embodiments, a clip member can be used to capture a leaflet in a folded configuration and at a position that avoids obstructing the corresponding coronary ostium. The clip member can have a biased state and a free state. In the biased state, ends of the clip member may be held at opposite ends of the clip member (e.g., having a substantially linear configuration). In the free state, ends of the clip member approach each other such that the clip member at least partially bounds a capture region. The clip member can be advanced in the biased state over a free end of the leaflet. One end of the clip member can be disposed on a distal side of the leaflet. The clip member can then be released from the biased state, thereby transitioning to the free state and capturing a part of the leaflet within the capture region. The new prosthetic heart valve can be subsequently installed.

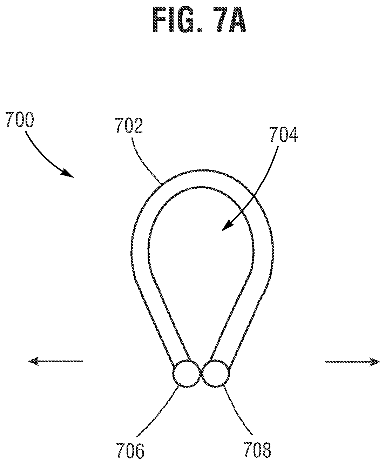

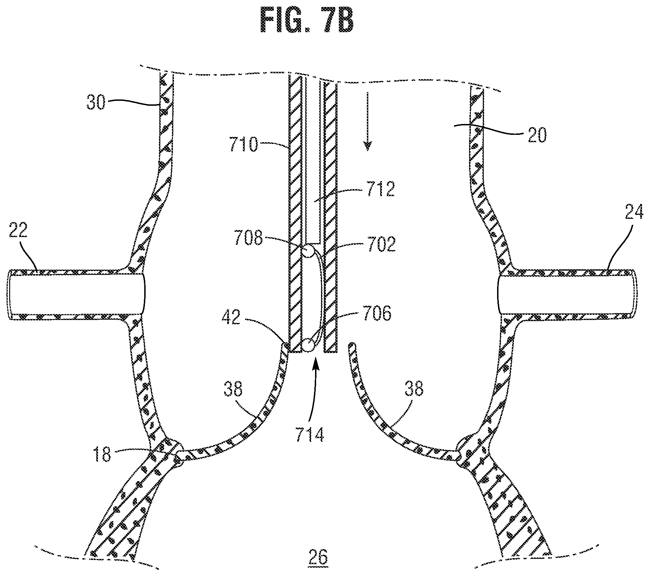

FIG. 7A illustrates an exemplary clip member in a free state, and FIGS. 7B-7D illustrate an exemplary method for folding and/or capturing a leaflet using the exemplary clip member. The clip member 700 can have ends 706, 708 connected together by an intermediate portion 702. In the free state illustrated in FIG. 7A, the clip member 700 has a tear-drop or Ω shape. The intermediate portion 702 adopts a curved configuration that defines a capture region 704 for receiving a leaflet therein. The ends 706, 708 can be in contact with each other or spaced from each other by a gap, which can be sized to be less than a thickness of the leaflet. The clip member 700 can be disposed in the biased state for loading into a delivery catheter and for initial positioning around part of the leaflet. In the biased state illustrated in FIG. 7B, the ends 706, 708 of the clip member 700 are pulled apart such that first end 706 is on a side of intermediate member 702 opposite from that of second end 708. In the biased state, the clip member 700 can adopt a substantially linear or arcuate configuration (e.g., bow-shaped).

The clip member 700 can be formed of a flexible material (e.g., spring metal) or a shape memory alloy that automatically transitions from the biased state to the free state upon release of a force that holds ends 706, 708 apart (e.g., when released from a delivery catheter). For example, the clip member can be formed of steel, cobalt chromium alloy, or nickel titanium alloy (e.g., Nitinol). In some embodiments, the clip member 700 can include a coating on one, some, or all portions thereof. For example, at least a portion of the clip member 700 can have a biocompatible surface coating. Alternatively or additionally, ends 706, 708 can be provided with a surface coating or surface treatment to improve retention to the leaflet once installed. For example, the surface coating or surface treatment may increase a coefficient of friction of ends 706, 708 as compared to a native state of the underlying material.

In some embodiments, a cross-sectional geometry of the clip member from end 706 to end 708 is substantially the same. For example, the clip member 700 can be formed of a substantially constant diameter flexible rod formed into the appropriate curved shape for the free state. In other embodiments, at least ends 706, 708 have a different shape and/or are formed of a different material from that of the intermediate portion 702. For example, the ends 706, 708 can be sized or shaped to assist in retaining the leaflet therebetween. In the illustrated example of FIG. 7A, ends 706, 708 are shaped as balls with diameters greater than a thickness of intermediate portion 702. The ball ends 706, 708 can act to pinch the leaflet therebetween once the clip member 700 transitions to the free state. Alternatively or additionally, the ends 706, 708 can be formed of a magnetic material. When disposed on the leaflet in the free state, ends 706, 708 can attract each other with the leaflet therebetween, which attraction force can improve retention of the clip member 700 to the leaflet. In another example, ends 706, 708 and/or portions of clip member 700 leading up to ends 706, 708 can be formed as extended flat regions that contact the leaflet when disposed thereon in the free state. The extended flat regions can increase the contact surface area, thereby improving the retention of the clip member 700 to the leaflet. Other shapes and configurations for the ends 706, 708 and/or the intermediate portion 702 are also possible according to one or more embodiments of the disclosed subject matter.

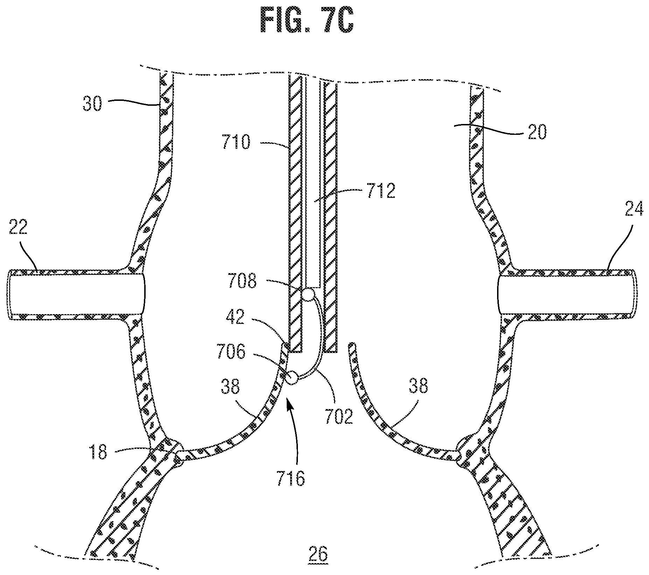

As shown in FIG. 7B, the clip member 700 can be disposed within a lumen of delivery catheter 710. The sidewalls of the lumen of the delivery catheter 710 can contact the ends 706, 708 and/or intermediate portion 702 to apply a biasing force that maintains the clip member 700 in the biased state. The delivery catheter 710 can be advanced from the ascending aorta 20 to be positioned with respect to one of the leaflet 38 of an existing valvular structure (e.g., a native aortic valve in the illustrated example, or a previously installed prosthetic heart valve in an unillustrated example). For example, the distal end 714 of the delivery catheter 710 can be disposed within the central gap between leaflets and adjacent to free end 42 of leaflet 38, as illustrated in FIG. 7B. The first end 706 can be extended from the distal end 714 of the delivery catheter 710 and into contact with a distal-side portion 716 of leaflet 38, as illustrated in FIG. 7C. Meanwhile, the sidewalls of the lumen of the delivery catheter 710 can continue to apply a biasing force between end 708 and intermediate portion 702 to maintain the biased state configuration. The positioning of the delivery catheter 710 with respect to the leaflet 38 (as shown in FIG. 7B) can occur at the same time, before, or after the extension of the first end 706 from the end 714 of the delivery catheter.

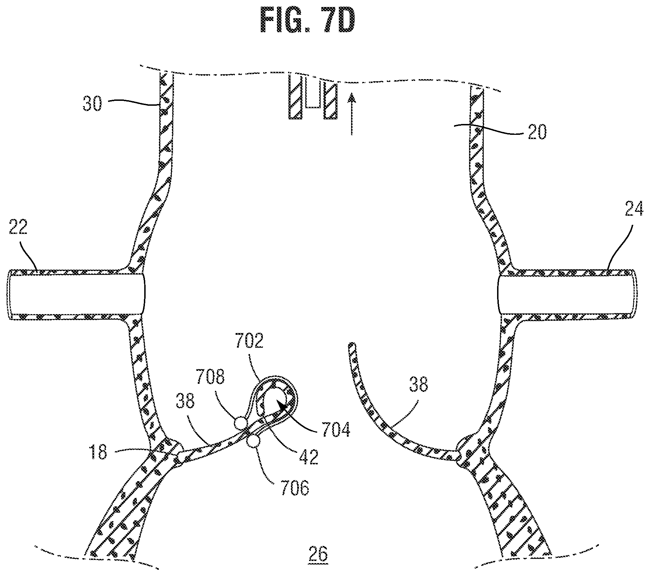

The clip member 700 can continue to be advanced from the end 714 of the delivery catheter 710 with the first end 706 contacting the distal side of the leaflet 38 until the second end 708 is released from the lumen of the delivery catheter 710. With the lumen no longer applying a biasing force, the clip member 700 is allowed to transition from the biased state to the free state, with ends 706, 708 approaching each other, as shown in FIG. 7D. The second end 708 thus curls distally toward the valve annulus 18 and is disposed on a proximal side of leaflet 38 at a location opposing the first end 706. As the clip member 700 transitions to the free state, the intermediate portion 702 contacts a proximal portion of the leaflet 38 (e.g., the portion adjacent to free end 42), thereby causing the proximal portion to fold upon itself and to be captured within capture region 704. The clip member 700 can be constructed such that the ends 706, 708 in the free state would contact each other or be separated by a gap that is less than a thickness of the leaflet 38. Thus, when the clip member 700 is disposed in the free state on the leaflet 38, a portion of the leaflet 38 between ends 706, 708 is pinched by the ends 706, 708 to retain the clip member 700 in place. The size of the clip member 700 and/or positioning thereof can be chosen such that the leaflet 38 is positioned distal to the ostium of coronary artery 22, even when pressed radially outward by subsequent mounting of a prosthetic heart valve within the existing valvular structure.

In some embodiments, the deployment of the clip member 700 from the delivery catheter 710 can be effected using a deployment member 712. The deployment member 712 may be a flexible rod that can be actuated to move axially within the delivery catheter 710. For example, the deployment member 712 can move distally within the lumen of the delivery catheter 710 to contact the second end 708 and push the clip member 700 from the distal end 714 of the catheter 710. In another example, the clip member 700 can be deployed by maintaining a position of the deployment member 712 with respect to existing valvular structure while retracting the catheter 710 proximally. In still another example, the clip member 700 can be deployed by a combination of movement of the catheter 710 (e.g., retracting proximally) and movement of the deployment member 712 (e.g., moving distally within the lumen of the delivery catheter).

The installation of the clip member 700 thus folds part of leaflet 38 and captures it distal to coronary artery 22, thereby preventing, or at least reducing the risk of obstruction of the ostium of coronary artery 22. If additional capture/folding of one or more leaflets 38 of the existing valvular structure is desired, for example, to prevent, or at least reduce the risk of, obstruction of the ostium of coronary artery 24, then the techniques of FIGS. 7B-7D can be repeated in a similar manner with respect to the next leaflet 38 using another clip member. The same delivery catheter 710 and/or deployment member 712 can be used to deploy the another clip member on the next leaflet 38. For example, two or more clip members 700 in the biased state can be serially disposed within the lumen of the same delivery catheter 710. The deployment member 712, in contact with a second end of a proximal clip member, can push the series of clip members 700 together so as to deploy each clip member sequentially from the end of the catheter 710.

When no further leaflet capture is desired, a new prosthetic heart valve in a crimped state can subsequently be advanced to the existing valvular structure with leaflets 38 captured by clip members 700 (such as shown in FIG. 4G). The new valve is disposed within the valvular structure and expanded, such that the captured leaflets 38 are disposed on an external surface of the new valve frame. However, the clip members 700 retain the capture leaflets 38 in a location distal to the coronary arteries 22, 24, thereby allowing blood to flow from the outflow end of the new prosthetic valve to the coronary arteries 22, 24 once the heart valve implantation is completed.

In some embodiments, the clip member can be plastically deformed to capture a leaflet in in a folded configuration and at a position that avoids obstructing the corresponding coronary ostium. The clip member can have first and second ends connected together by an intermediate portion. In an open configuration of the clip member, the first and second ends can be spaced apart from each other by a gap. In the closed configuration of the clip member, the gap can be reduced or eliminated. The clip member can be positioned in the open configuration such that a free end of the leaflet extends through the gap. The clip member can be move toward a distal portion (e.g., cusp portion) of the leaflet to cause folding of the leaflet. An actuator can then be used to apply a compressive force to the clip member that causes the clip member to plastically deform (e.g., bend) to the closed configuration. At least a folded part of the leaflet can be captured within a region enclosed by the clip member in the closed configuration. The new prosthetic heart valve can be subsequently installed.

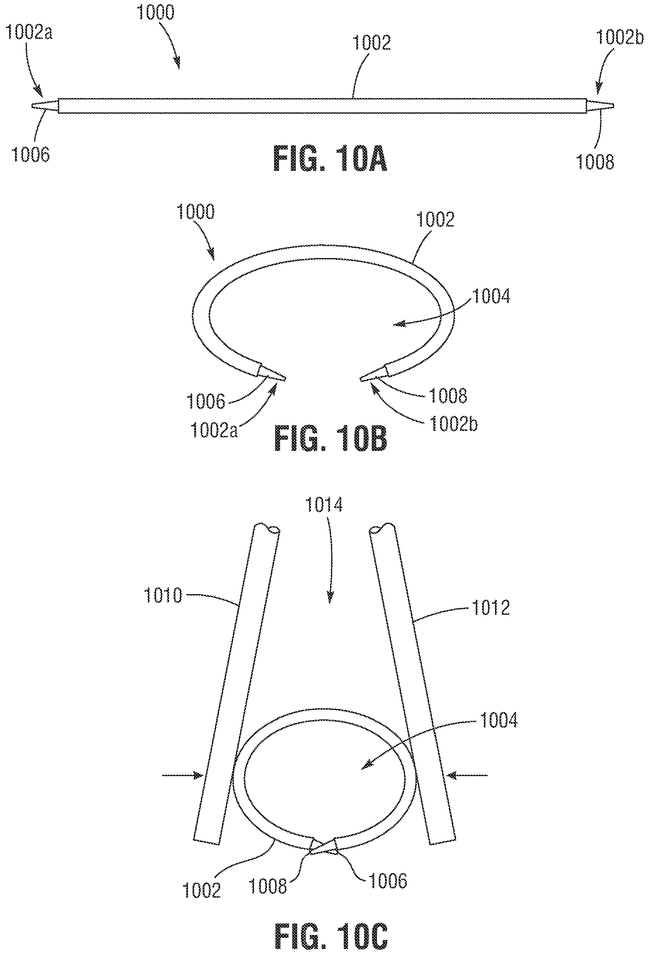

For example, FIG. 10A illustrates a clip member 1000 in an initial configuration, FIG. 10B illustrates the clip member 1000 in an open configuration, and FIG. 10C illustrates the clip member 1000 in a closed configuration. The clip member 1000 can have a first end 1002a and a second end 1002b connected together by an intermediate portion 1002. In the initial configuration illustrated in FIG. 10A, the clip member 1000 has a substantially linear shape, with the first and second ends being on opposite ends of intermediate portion 1002 with respect to a longitudinal axis thereof. Such a configuration may be useful, for example, for delivering the clip member 1000 to an implantation location via a delivery catheter or sheath.

Once delivered to the implantation location, the clip member 1000 may be plastically deformed to the open configuration of FIG. 10B, for example, by bending the intermediate portion 1002 such that ends 1002a, 1002b face each other across a gap 1016 (e.g., to have a substantially C-shape). The clip member 1000 in the open configuration thus defines a partially-enclosed area or capture region 1004. Alternatively, in some embodiments, the clip member 1000 may be initially formed in the open configuration of FIG. 10B, for example, by casting, molding, or otherwise forming the intermediate portion 1002 to have the C-shape without bending from the linear configuration of FIG. 10A.

To transition the clip member 1000 to the closed configuration of FIG. 10C, the clip member 1000 is disposed in an area 1014 between a first arm 1010 and a second arm 1012 of an actuator. The actuator applies a compressive force that plastically deforms the clip member such that the gap 1016 between the first and second ends is eliminated or at least reduced (e.g., less than a thickness of the leaflet), thereby further enclosing capture region 1004 to capture a part of the leaflet therein. The actuator can be, for example, tweezers, surgical tongs, or a scissor-style actuator (e.g., similar to actuator 612 illustrated in FIG. 6A). However, other actuators are also possible according to one or more contemplated embodiments. Indeed, the actuator can be any type of actuator capable of operating within the ascending aorta of a patient and of applying a compressive force to the clip member. For example, the arms 1010, 1012 can be coupled to opposite ends of the clip member 1000 and a sheath can be advanced over the arms from a proximal end thereof toward a distal end thereof, thereby urging the arms together to apply the compressive force to the clip member.

The clip member 1000 can be formed of a material having sufficient rigidity to maintain its shape after being plastically deformed. For example, the clip member 1000 can comprise a metal, metal-alloy, or any combination thereof, such as steel or cobalt chromium alloy. In some embodiments, the clip member 1000 can include a coating on one, some, or all portions thereof. For example, at least a portion of the clip member 1000 can have a biocompatible surface coating. Alternatively or additionally, ends 1002a, 1002b (e.g., tip 1006 and/or tip 1008) can be provided with a surface coating or surface treatment to improve retention to the leaflet once installed. For example, the surface coating or surface treatment may increase a coefficient of friction of tips 1006, 1008 as compared to a native state of the underlying material.

In some embodiments, a cross-sectional geometry of at least the intermediate portion between ends 1002a, 1002b can be substantially the same. For example, the clip member 1000 can be formed of a substantially constant diameter rod plastically deformed from the linear configuration of FIG. 10A to the open configuration of FIG. 10B. In some embodiments, at least ends 1002a, 1002b can have a different shape and/or are formed of a different material from that of the intermediate portion 1002. For example, the ends 1002a, 1002b can be sized or shaped to assist in retaining the leaflet therebetween. In the illustrated example of FIGS. 10A-10C, the first end 1002a has a first tip 1006 and the second end 1002b has a second tip 1008. Each tip 1006, 1008 can taper in a direction away from the intermediate portion 1002 to a sharp point or at least a narrowed-diameter end, so as to be able to pierce through the leaflet when contacting therewith. Alternatively, in some embodiments, tips 1006, 1008 can be replaced with ball ends that act to pinch the leaflet therebetween when the clip member 1000 is in the closed configuration, similar to the ball ends illustrated in FIG. 7A. Alternatively or additionally, the tips 1006, 1008 and/or ends 1002a, 1002b can be formed of a magnetic material. When disposed on the leaflet in the open configuration, the magnetic material can attract each other with the leaflet therebetween, which attraction force can improve retention of the clip member 1000 to the leaflet. Further shapes and configurations for the ends 1002a, 1002b and/or the intermediate portion 1002 are also possible according to one or more embodiments of the disclosed subject matter.

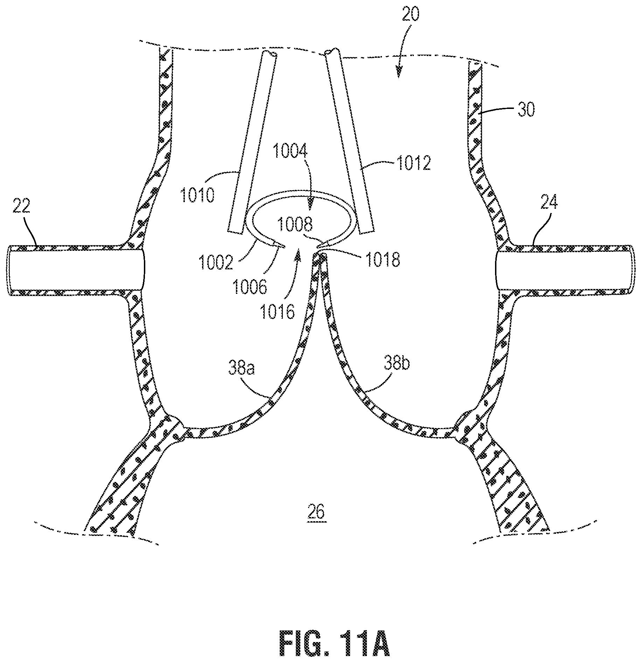

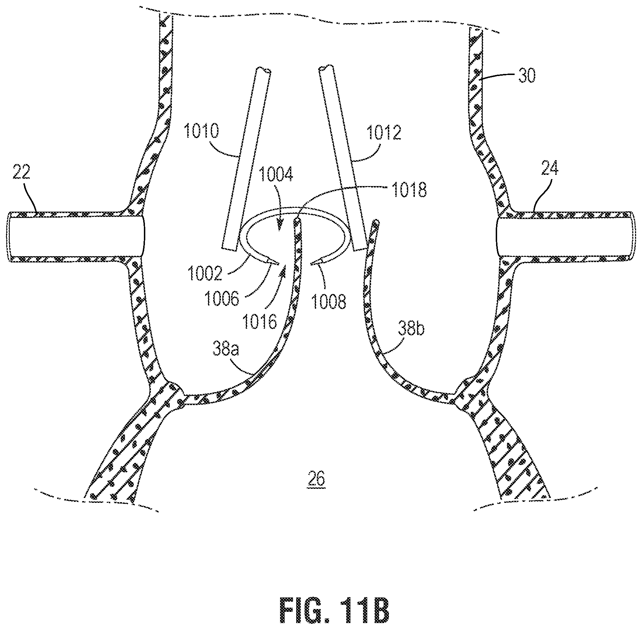

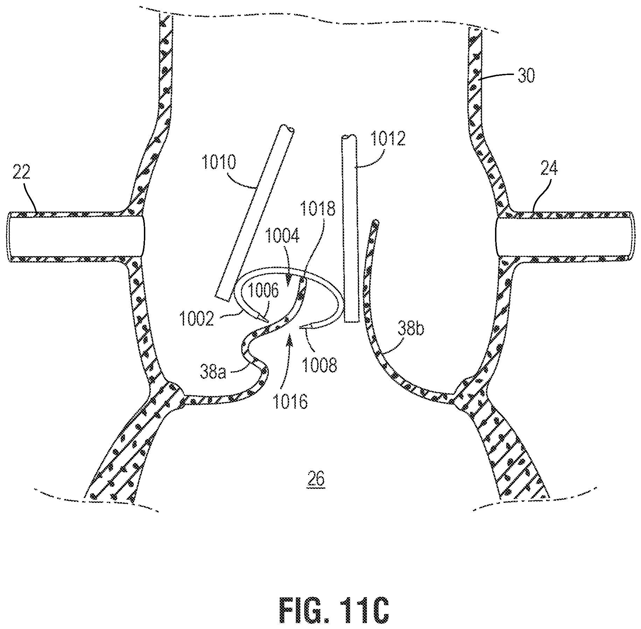

As shown in FIG. 11A, actuator arms 1010, 1012 can be used to advance the clip member in the open configuration from the ascending aorta 20 so as to be positioned with respect to one of the leaflet 38a of an existing valvular structure (e.g., a native aortic valve in the illustrated example, or a previously installed prosthetic heart valve in an unillustrated example). For example, the free end 1018 of leaflet 38a can be arranged to extend through gap 1016 between the first tip 1006 and the second tip 1008 into the capture region 1004, as shown in FIG. 11B. The actuator arms 1010, 1012 can then be used to further advance the clip member along the leaflet 38a, as shown in FIG. 11C. For example, as the clip member is moved toward a distal portion of the leaflet 38a (e.g., a root or cusp region of the leaflet, or an annulus of the valvular structure), the intermediate portion 1002 of the clip member abuts the free end 1018 of the leaflet 38a and causes it to fold upon itself, such that folded portion of leaflet 38a is gathered within gap 1016.

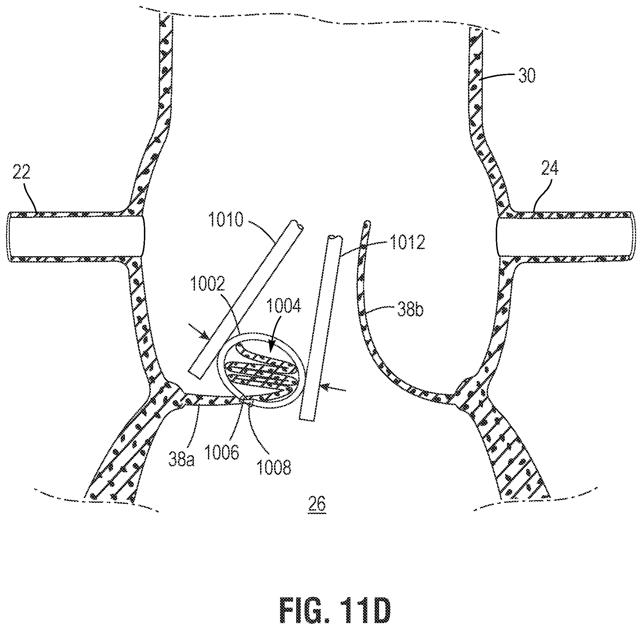

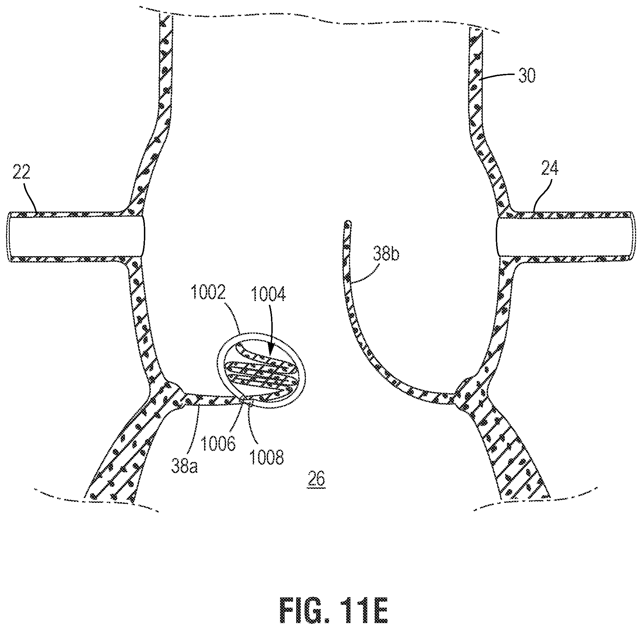

The amount of clip member advancement can be chosen such that the folded leaflet 38a is positioned distal to the ostium of coronary artery 22, even when pressed radially outward by subsequent mounting of a prosthetic heart valve within the existing valvular structure. Once the clip member has been sufficiently advanced over the leaflet, the actuator can be actuated to apply a compressive force between arms 1010, 1012, thereby plastically deforming the clip member to the closed configuration, as shown in FIG. 11D. The tips 1006, 1008 of the clip member thus move toward each other and pierce, or at least clamp, a portion of the leaflet 38a therebetween. The plastic deformation of the clip member can be effective to lock the tips 1006, 1008 in position within respect to the leaflet 38a. The actuator can then be released from the clip member and can be used to subsequently install a clip member on another leaflet or otherwise retrieved from the patient. After removal of the actuator, the clip member remains behind in place on the folded leaflet 38a, as shown in FIG. 11E. The plastic deformation of the clip member can thus ensure that the part of the leaflet 38a captured within region 1004 does not unfold during or after installation of the prosthetic heart valve.

It should be understood that only a portion of the actuator arms 1010, 1012 are shown in FIGS. 11A-11D for clarity of illustration. However, in practical embodiments, the actuator would include additional portions not illustrated, such as a hinge section (e.g., for scissor-style actuators), a joint section (e.g., for tweezer or surgical tongs), and/or a delivery catheter or sheath (e.g., for a sheath advanced over the arms to provide a force urging the arms together). Moreover, one or more delivery catheters or sheaths can be provided to deliver the clip member and the actuator to the ascending aorta, and/or for operation of the actuator within the ascending aorta.

The installation of the clip member thus folds part of leaflet 38a and captures it distal to coronary artery 22, thereby preventing, or at least reducing the risk of obstruction of the ostium of coronary artery 22. If additional capture/folding of one or more leaflets 38 of the existing valvular structure is desired, for example, to prevent, or at least reduce the risk of, obstruction of the ostium of coronary artery 24, then the techniques of FIGS. 11A-11E can be repeated in a similar manner with respect to the next leaflet 38 using another clip member and the same actuator (e.g., arms 1010, 1012) or a different actuator. Although not discussed in detail above, manipulation of the clip member 1000 and/or the actuator within the patient's anatomy, actuation of the actuator to plastically deform clip member 1000, and/or decoupling of the actuator from the clip member 1000 may be performed using any tools employed in laparoscopic and/or transcatheter heart surgeries.

When no further leaflet capture is desired, a new prosthetic heart valve in a crimped state can subsequently be advanced to the existing valvular structure with leaflets 38 captured by clip member 1000. The new valve is disposed within the valvular structure and expanded, such that the captured leaflets 38 are disposed on an external surface of the new valve frame. However, the clip members retain the captured leaflets 38 in locations distal to the coronary arteries 22, 24, thereby allowing blood to flow from the outflow end of the new prosthetic valve to the coronary arteries 22, 24 once the heart valve implantation is completed.

In some embodiments, a tool can be used to temporarily fold a leaflet below the ostia of the coronary artery and hold it in place until a prosthetic heart valve can be positioned within the existing valvular structure and at least partially expanded therein. As the prosthetic heart valve expands, the folded leaflet is pushed by the valve frame wall toward and into contact with the surrounding anatomy (e.g., the aortic wall) or pre-existing structure (e.g., the valve frame wall of a previously implanted prosthetic valve). Prior to full expansion of the prosthetic heart valve (e.g., before a size of an annular region between the valve frame and the surrounding structure would prevent removal of the tool), the portion of the tool coupled to the leaflet can be pulled away therefrom. Since the leaflets have been positioned distally and folded, the risk of curtaining of the coronary ostia during valve expansion can be reduced.

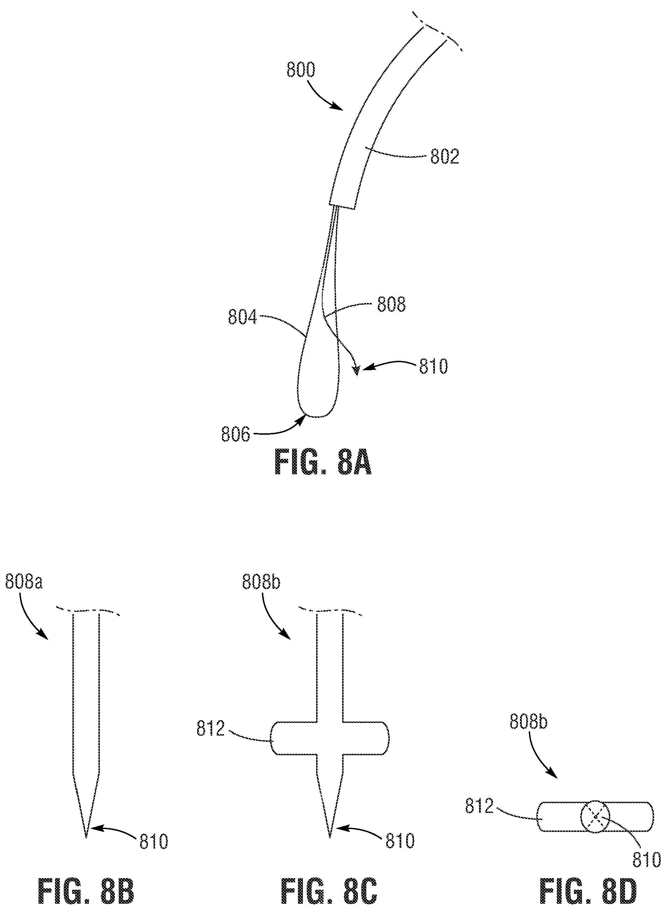

For example, FIG. 8A illustrates an exemplary leaflet folding tool 800 that can be used to temporarily fold a leaflet of an existing valvular structure. The tool 800 comprises a delivery catheter or sheath 802 with a distal end configured to be disposed within an ascending aorta of a patient. The sheath 802 can contain at least two members - a positioning member 804 and a leaflet engagement member 808. Each of the members 804, 808 can be independently maneuvered within sheath 802 and extending from sheath 802, for example, via operation of a handle (not shown) at the proximal region of the sheath 802 by an operator. The sheath 802 in the illustrated embodiment is in the form of a shaft, although in other embodiments, the sheath can include multiple shafts, which can be disposed adjacent to each other within the same sheath.

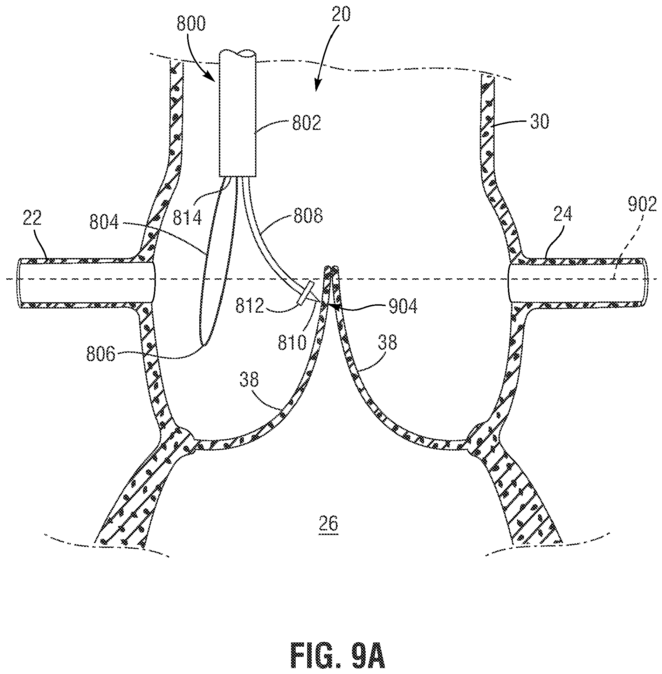

Each of the members 804, 808 can be extended from the distal end of the sheath 802 to interact with the existing valvular structure. For example, as shown in FIGS. 9A-9F, the positioning member 804 can be configured for insertion into a pocket or sinus 906 between a leaflet 38 of the existing valvular structure and the frame 12 (or the aortic wall 30 when dealing with the native valve), in order to guide placement and positioning of the leaflet engagement member 808 with respect to leaflet 38. Each of the members 804, 808 can be a pre-shaped wire or cable, for example, a wire formed of a shape memory material, such as Nitinol. Thus, as the members 804, 808 are advanced out of the distal end of the sheath, end portions thereof can adopt their pre-determined shape. Alternatively, positioning member 804 and/or leaflet engagement member 808 can be formed of other materials, such as a metal (e.g., steel, titanium, etc.), metal alloy (e.g., cobalt chromium alloy, etc.), plastic, or any combination thereof.

In some embodiments, at least a distal-most end portion 806 of the positioning member 804 is constructed to be atraumatic (e.g., blunt or otherwise lacking sharp edges) to avoid damage to the surrounding anatomy during operation. For example, the positioning member 804 can have a contoured end portion 806 having a circular shape, oval shape (e.g., spoon-shaped), elliptical shape, C-shape, J-shape, or any other arcuate shape, such that an area of contact between the end portion 806 and a base (e.g., cusp portion) of the leaflet can be increased. In some embodiments, the positioning member 804 can be formed from a plurality of wires, for example, as a pair of wires that are bent laterally to converge to contact or attach to each other at end portion 806. Alternatively, in some embodiments, the positioning member comprises a catheter or sheath with an atraumatic distal end.

The leaflet engagement member 808 can comprise a spiked tip 810 at an axial end thereof, which is constructed to penetrate or otherwise grasp a leaflet. In some embodiments, the spiked tip 810 simply includes a sharp point or spike, for example, as with member 808a illustrated in the detail view of FIG. 8B. The sharp point can be used to pierce a portion of the leaflet in order to engage therewith. In other embodiments, the tip can have a barbed configuration that prevents or at least resists further insertion of the tip through the leaflet, so as to assist in pushing the leaflet distally. For example, in the detail views of FIGS. 8C-8D, leaflet engagement member 808b has a barbed configuration with laterally-extending base 812 being spaced proximally from the spiked tip 810 along an axial direction of the member 808b. Alternatively, the barbed configuration can comprise an annular base (e.g., a radially-extending base) instead of the laterally-extending base 812.

FIGS. 9A-9C show various stages for using the leaflet folding tool 800 to distally position a leaflet 38 of the existing valvular structure to avoid coronary artery obstruction by subsequent implantation of a prosthetic heart valve. Delivery shaft 802 can be advanced to the existing valvular structure from the ascending aorta 20. The positioning member 804 and the leaflet engagement member 808 may be retained within the shaft 802 prior to reaching the existing valvular structure. Once the shaft 802 reaches the existing valvular structure (e.g., the distal end 814 is positioned near the coronary artery 22), the positioning member 804 can be advanced from the distal end 814 of the shaft 802 toward, but spaced from, a base 906 of the leaflet 38 (e.g., the cusp floor of the leaflet). Simultaneous with the advancement of the positioning member 804 or sequential thereto (e.g., before or after), the leaflet engagement member 808 can be advanced from the distal end 814 of the shaft toward a proximal portion 904 of the leaflet 38, as shown in FIG. 9A.

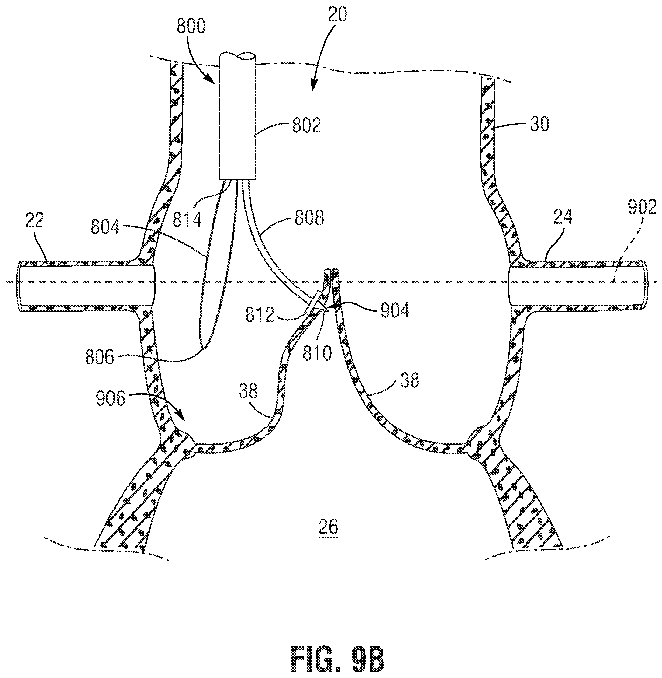

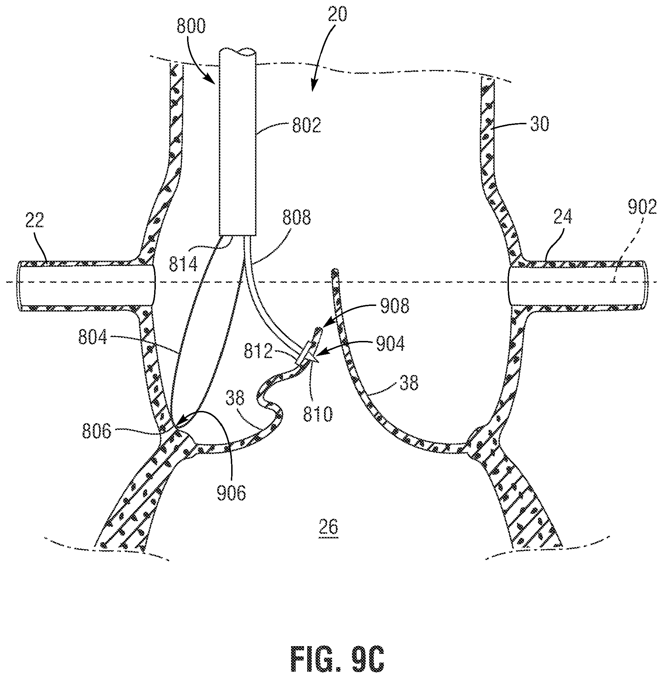

The leaflet engagement member 808 can be pushed into contact with the leaflet portion 904, such that the spiked tip 810 penetrates therethrough (e.g., from a proximal side thereof to a distal side thereof), as shown in FIG. 9B. The lateral base 812 is brought into contact with the proximal side of the leaflet portion 904 to restrict further insertion of the spiked tip 810. The lateral base 812 also increases the surface contact area to assist in pushing the leaflet distally. In particular, the leaflet engagement member 808 and the positioning member 804 can be advanced together (e.g., by moving delivery shaft 802 distally, by simultaneously advancing members 804, 808 distally out of shaft 802, or by any combination thereof), such that the engagement member 808 pushes the free end 908 of the leaflet away from the plane 902 of the coronary ostia, effectively folding the leaflet, as shown in FIG. 9C. The advancement of both members 804, 808 can continue until the positioning member 804 reaches the base portion 906 of the leaflet 38. With the leaflet 38 held in this folded position, a prosthetic heart valve can be subsequently implanted, whereby the folded leaflet 38 reduces the risk of obstruction the ostium of coronary artery 22 after valve implantation.

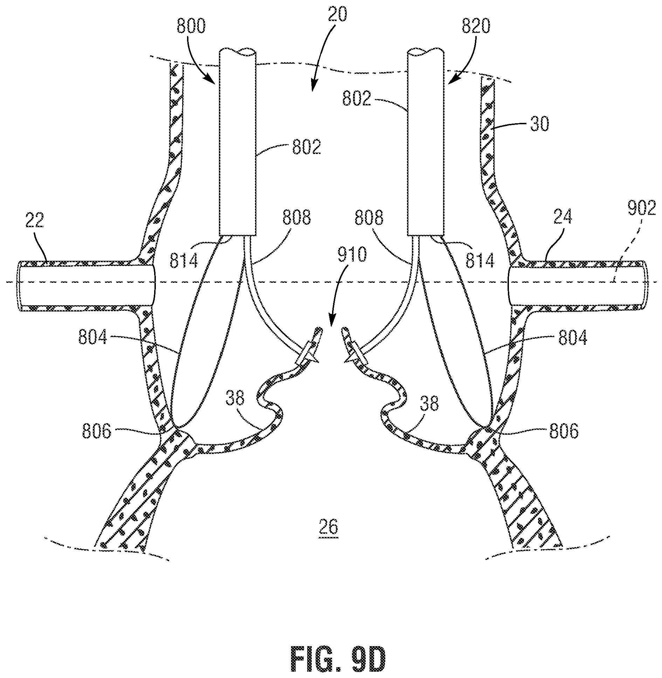

In some embodiments, at least two of the leaflets of the existing valvular structure can be subjected to folding, for example, using a respective leaflet folding tool. For example, a leaflet folding tool can be provided for each leaflet (e.g., three folding tools for three leaflets). If folding of additional leaflets of the existing valvular structure is desired, for example, to prevent, or at least reduce the risk of, obstruction of the ostium of coronary artery 24, then the techniques of FIGS. 9A-9C can be repeated in a similar manner with respect to the next leaflet 38 using a separate folding tool 820, for example, as shown in FIG. 9D. In such repetition, the portion 904 of each leaflet 38 folded by the respective tool 800, 820 can be circumferentially aligned with the ostium of coronary artery 22 or coronary artery 24 (e.g., aligned or substantially aligned along a same radial vector extending from a center of the existing valvular structure, as viewed from the ascending aorta 20).

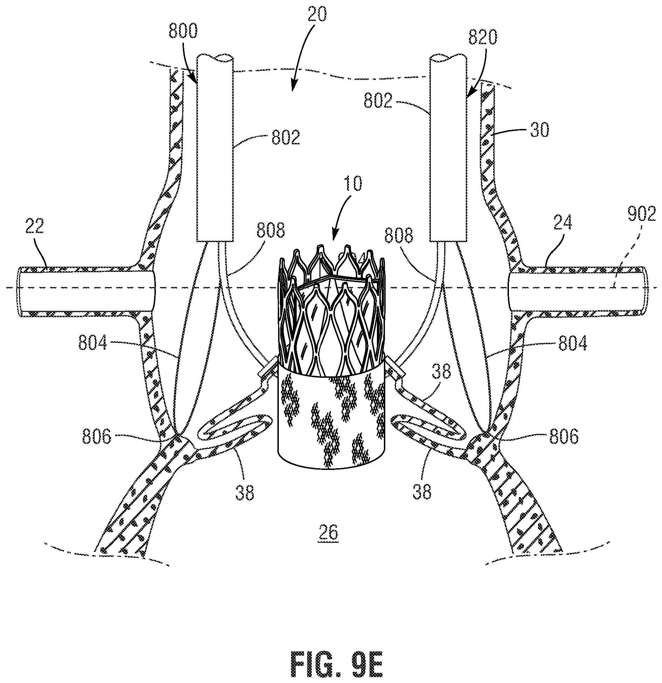

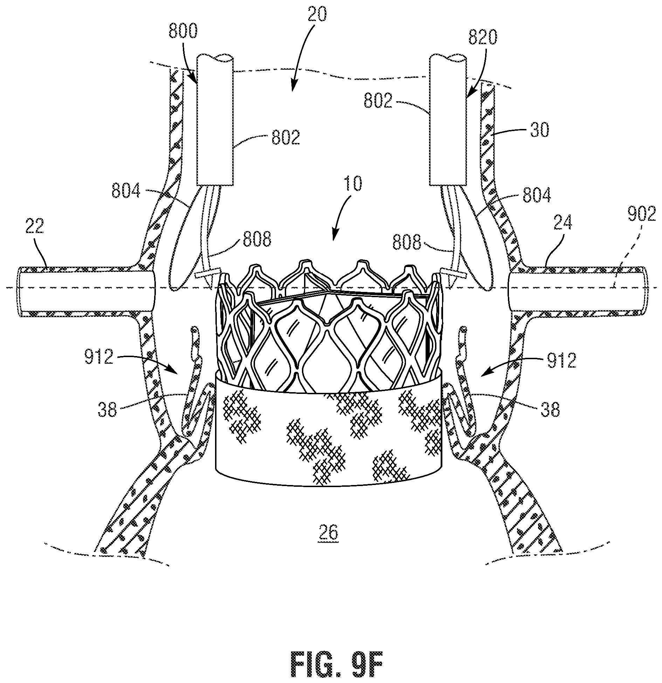

When no further leaflet folding is desired, a new prosthetic heart valve in a crimped state can subsequently be advanced to the existing valvular structure with captured leaflets 38. For example, as depicted in FIG. 9E, a new prosthetic valve 10 (which can be the valve 10 of FIG. 2A or a different prosthetic valve) can be positioned in region 910 between free ends of the leaflets 38, as shown in FIG. 9E. The new prosthetic valve 10 is then partially expanded such that the folded leaflets 38 are disposed in an annular region 912 between the external circumferential surface of the new valve frame and the surrounding structures (e.g., the aortic wall), as shown in FIG 9F. Prior to full expansion of the valve 10, the leaflet engagement members 808 can be removed from the respective leaflets 38 (e.g., by retracting the leaflet engagement members proximally) and the members 804, 808 removed from annular region 912, thereby allowing the valve 10 to fully expand toward the aortic wall 30. Once disengaged from the leaflets 38, the leaflet folding tools 800, 820 can be removed from the ascending aorta, for example, by retracting members 804, 808 into the respective sheath 802 and removing from the vasculature of the patient. The folded leaflets are thus pressed sideways against the aortic wall 30 by the fully-expanded frame of prosthetic heart valve 10 at a location distal to the plane 902 of the coronary ostia, thereby reducing the risk of obstruction of the coronary arteries 22, 24 and allowing blood to flow from the outflow end of the new prosthetic valve to the coronary arteries 22, 24 once the heart valve implantation is completed.

In any of the above noted examples, any of the delivery catheters or sheaths, locking devices, folding tools, and/or clip members can be provided from the ascending aorta to the existing valvular structure via any transcatheter aortic access route, such as but not limited to transfemoral, transaxillary, transaortic, transapical, transcarotid, transseptal, transcaval, subclavian, radial, or carotid approaches.

In any of the above noted examples, any of the delivery catheters or sheaths, locking devices, folding tools, and/or clip members can be used to capture/fold leaflets of the native valvular structure of another of the native heart valves, for example, pulmonary, tricuspid, or mitral valves, or prosthetic heart valves previously implanted therein. It should be noted that any of the devices or techniques described herein in connection with folding or otherwise modifying the position of a native leaflet to avoid or minimize blockage of the coronary ostia can be applied to one or more leaflets of a previously implanted prosthetic heart valve prior to or while implanting a new prosthetic heart valve in the previously implanted prosthetic heart valve in a valve-in-valve procedure. Further, any of the delivery catheters or sheaths, locking devices, folding tools, and/or clip members can thus be configured to be delivered via other blood vessels to the heart. For example, to access the tricuspid or pulmonary positions, the delivery catheters or sheaths, locking devices, folding tools, and/or clip members can be delivered via the inferior and superior vena cava. For accessing the mitral position, the delivery catheters or sheaths, locking devices, folding tools, and/or clip members can be delivered via a transseptal procedure, for example, by advancing through the inferior or superior vena cava and across through the atrial septum. Alternatively, for accessing the mitral position, the delivery catheters or sheaths, locking devices, folding tools, and/or clip members can be delivered via a transapical approach, for example, by advancing through the wall of the left ventricle at the base of the heart.

In any of the above noted examples, the capture/folding of leaflets in the existing valvular structure can be performed as part of, before, or after a balloon annular valvuloplasty procedure on the existing valvular structure, for example, to prepare an existing heart valve (e.g., native aortic valve or previously implanted prosthetic valve) for subsequent implantation of a new prosthetic valve. Alternatively, in any of the above noted examples, the capture/folding of leaflets in the existing valvular structure can be performed as part of or preceding a transcatheter aortic valve implantation (TAVI) or transcatheter aortic valve replacement (TAVR) procedure, for example, to replace a native aortic valve or to replace a failing prosthetic valve (e.g., ViV procedure). Indeed, while the examples of FIGS. 3A-11E specifically illustrate capture/folding of leaflets in a native aortic valve, it should be readily understood that the same techniques can be applied to previously-implanted prosthetic heart valves, for example, prior to or simultaneous with the mounting of a new prosthetic heart valve within the previously-implanted heart valve. Accordingly, embodiments of the disclosed subject matter are not limited to the particular illustrations.

Where not otherwise expressly noted above, components can be formed of any type of biocompatible material of sufficient strength or flexibility for use in the particular application. Such materials can include, but are not limited to, metals or metal alloys such as surgical steel, titanium, cobalt chromium alloy. and nickel titanium alloy (nitinol), and polymers such as polyurethane, polytetrafluorethylene, and polyethersulfone.

### Conclusion

In view of the many possible embodiments to which the principles of the disclosed technology may be applied, it should be recognized that the illustrated embodiments are only preferred examples and should not be taken as limiting the scope of the disclosed technology. Rather, the scope is defined by the following claims.

## Claims

1. A prosthetic heart valve (10) comprising:
an annular frame (12) that is expandable from a crimped state to a deployed state;
a first valvular structure formed by a leaflet assembly, the leaflet assembly comprising a plurality of leaflets (14), the first valvular structure being disposed within and attached to the annular frame (12); and
a leaflet capture member (304) disposed along an exterior of the annular frame (12) and coupled at opposite ends thereof to the annular frame (12), the leaflet capture member (304) extending along an axial direction of the annular frame (12), the leaflet capture member (304) comprising a distal portion (318), a proximal portion, and an intermediate portion (312) between and coupled to the distal (318) and proximal portions by respective connecting portions (310, 314),
wherein the leaflet capture member (304) is configured to change shape upon expansion of the annular frame (12) to the deployed state so as to capture a free end (42) of a leaflet (38) of an existing second valvular structure between the intermediate portion (312) and the distal portion (318).

2. The prosthetic heart valve (10) of claim 1, further comprising additional leaflet capture members (304) disposed at different locations along a circumference of the annular frame (12).

3. The prosthetic heart valve (10) of any preceding claim, wherein each leaflet capture member (304) comprises a substantially straight bar extending along the axial direction prior to expansion of the annular frame (12).

4. The prosthetic heart valve (10) of any preceding claim, wherein at least the proximal portion of each leaflet capture member (304) has an open cell structure that allows blood to flow therethrough.

5. The prosthetic heart valve (10) of any preceding claim, wherein at least one of the connecting portions (310, 314) comprises a weakened region.

6. The prosthetic heart valve (10) of any preceding claim, wherein at least one of the connecting portions (310, 314) comprises a region with a narrowed cross-section compared to the distal portion, the proximal portion, and/or the intermediate portion.

7. The prosthetic heart valve (10) of any preceding claim, wherein at least one of the connecting portions (310, 314) comprises a notched or grooved region.

8. The prosthetic heart valve (10) of any preceding claim, wherein the leaflet capture member (304) is formed of metal or a biocompatible polymer.

9. The prosthetic heart valve (10) of any preceding claim, wherein the leaflet capture member (304) is configured such that:
prior to expansion of the annular frame (12), a first connecting portion (310) that couples the proximal portion to the intermediate portion (312) is disposed proximal to a second connecting portion (31) that couples the distal portion (318) to the intermediate portion (312); and
after expansion of the annular frame (12), the first connecting portion (310) is disposed distal to the second connecting portion (314).

## Patentansprüche

1. Herzklappenprothese (10), umfassend:
einen ringförmigen Rahmen (12), der aus einem gecrimpten Zustand in einen abgelegten Zustand expandiert werden kann,
eine durch eine Klappensegelbaugruppe gebildete erste Klappenstruktur, wobei die Klappensegelbaugruppe eine Vielzahl von Klappensegeln (14) umfasst, wobei die erste Klappenstruktur in dem ringförmigen Rahmen (12) angeordnet und an ihm angebracht ist, und
in Klappensegelerfassungsglied (304), das entlang einer Außenseite des ringförmigen Rahmens (12) angeordnet und an gegenüberliegenden Enden davon an den ringförmigen Rahmen (12) gekoppelt ist, wobei sich das Klappensegelerfassungsglied (304) entlang einer axialen Richtung des ringförmigen Rahmens (12) erstreckt, wobei das Klappensegelerfassungsglied (304) einen distalen Abschnitt (318), einen proximalen Abschnitt und einen Zwischenabschnitt (312) umfasst, der zwischen dem distalen (318) und dem proximalen Abschnitt liegt und über jeweilige Verbindungsabschnitte (310, 314) an diese gekoppelt ist,
wobei das Klappensegelerfassungsglied (304) dazu ausgestaltet ist, bei der Expansion des ringförmigen Rahmens (12) in den abgelegten Zustand seine Form zu ändern, um ein freies Ende (42) eines Klappensegels (38) einer vorliegenden zweiten Klappenstruktur zwischen dem Zwischenabschnitt (312) und dem distalen Abschnitt (318) zu erfassen.

2. Herzklappenprothese (10) nach Anspruch 1, ferner umfassend zusätzliche Klappensegelerfassungsglieder (304), die an verschiedenen Stellen entlang eines Umfangs des ringförmigen Rahmens (12) angeordnet sind.

3. Herzklappenprothese (10) nach einem der vorhergehenden Ansprüche, wobei jedes Klappensegelerfassungsglied (304) einen im Wesentlichen geraden Stab umfasst, der sich vor der Expansion des ringförmigen Rahmens (12) entlang der axialen Richtung erstreckt.

4. Herzklappenprothese (10) nach einem der vorhergehenden Ansprüche, wobei mindestens der proximale Abschnitt jedes Klappensegelerfassungsglieds (304) eine offene Zellenstruktur aufweist, die gestattet, dass Blut dort hindurchfließt.

5. Herzklappenprothese (10) nach einem der vorhergehenden Ansprüche, wobei mindestens einer der Verbindungsabschnitte (310, 314) einen geschwächten Bereich umfasst.

6. Herzklappenprothese (10) nach einem der vorhergehenden Ansprüche, wobei mindestens einer der Verbindungsabschnitte (310, 314) einen Bereich mit einem im Vergleich zu dem distalen Abschnitt, dem proximalen Abschnitt und/oder dem Zwischenabschnitt verengten Querschnitt umfasst.

7. Herzklappenprothese (10) nach einem der vorhergehenden Ansprüche, wobei mindestens einer der Verbindungsabschnitte (310, 314) einen gekerbten oder genuteten Bereich umfasst.

8. Herzklappenprothese (10) nach einem der vorhergehenden Ansprüche, wobei das Klappensegelerfassungsglied (304) aus Metall oder einem biokompatiblen Polymer gebildet ist.

9. Herzklappenprothese (10) nach einem der vorhergehenden Ansprüche, wobei das Klappensegelerfassungsglied (304) so ausgestaltet ist, dass:
ein erster Verbindungsabschnitt (310), der den proximalen Abschnitt an den Zwischenabschnitt (312) koppelt, vor der Expandierung des ringförmigen Rahmens (12) proximal zu einem zweiten Verbindungsabschnitt (31) angeordnet ist, der den distalen Abschnitt (318) an den Zwischenabschnitt (312) koppelt, und
der erste Verbindungsabschnitt (310) nach der Expansion des ringförmigen Rahmens (12) distal zu dem zweiten Verbindungsabschnitt (314) angeordnet ist.

## Revendications

1. Valvule cardiaque prothétique (10) comprenant :
un cadre annulaire (12) qui est extensible d'un état serti à un état déployé ;
une première structure valvulaire formée par un ensemble de feuillets, l'ensemble de feuillets comprenant une pluralité de feuillets (14), la première structure valvulaire étant disposée à l'intérieur du cadre annulaire (12) et fixée à celui-ci ; et
un élément de capture de feuillet (304) disposé le long d'un extérieur du cadre annulaire (12) et couplé à ses extrémités opposées au cadre annulaire (12), l'élément de capture de feuillet (304) s'étendant le long d'une direction axiale du cadre annulaire (12), l'élément de capture de feuillet (304) comprenant une partie distale (318), une partie proximale et une partie intermédiaire (312) située entre les parties distale (318) et proximale par des parties de connexion respectives (310, 314) et couplée à celles-ci,
l'élément de capture de feuillet (304) étant configuré pour changer de forme lors de l'expansion du cadre annulaire (12) à l'état déployé de manière à capturer une extrémité libre (42) d'un feuillet (38) d'une seconde structure valvulaire existante entre la partie intermédiaire (312) et la partie distale (318).

2. Valvule cardiaque prothétique (10) selon la revendication 1, comprenant en outre des éléments de capture de feuillet supplémentaires (304) disposés à différents emplacements le long d'une circonférence du cadre annulaire (12).

3. Valvule cardiaque prothétique (10) selon l'une quelconque des revendications précédentes, chaque élément de capture de feuillet (304) comprenant une barre sensiblement droite s'étendant le long de la direction axiale avant l'expansion du cadre annulaire (12).

4. Valvule cardiaque prothétique (10) selon l'une quelconque des revendications précédentes, au moins la partie proximale de chaque élément de capture de feuillet (304) ayant une structure à cellules ouvertes qui permet au sang de s'écouler à travers elle.

5. Valvule cardiaque prothétique (10) selon l'une quelconque des revendications précédentes, au moins l'une des parties de connexion (310, 314) comprenant une région affaiblie.

6. Valvule cardiaque prothétique (10) selon l'une quelconque des revendications précédentes, au moins l'une des parties de connexion (310, 314) comprenant une région avec une section transversale rétrécie par rapport à la partie distale, la partie proximale et/ou la partie intermédiaire.

7. Valvule cardiaque prothétique (10) selon l'une quelconque des revendications précédentes, au moins l'une des parties de connexion (310, 314) comprenant une région entaillée ou rainurée.

8. Valvule cardiaque prothétique (10) selon l'une quelconque des revendications précédentes, l'élément de capture de feuillet (304) étant formé de métal ou d'un polymère biocompatible.

9. Valvule cardiaque prothétique (10) selon l'une quelconque des revendications précédentes, l'élément de capture de feuillet (304) étant configuré de telle sorte que :
avant l'expansion du cadre annulaire (12), une première partie de connexion (310) qui couple la partie proximale à la partie intermédiaire (312) est disposée à proximité d'une seconde partie de connexion (31) qui couple la partie distale (318) à la partie intermédiaire (312) ; et
après expansion du cadre annulaire (12), la première partie de connexion (310) est disposée de manière distale par rapport à la seconde partie de connexion (314).
